# EUROPEAN PATENT APPLICATION

(11) **EP 4 342 408 A1**
(43) Date of publication of application: **27.03.2024**
(21) Application number: 22208402.2
(22) Date of filing: 18.11.2022
(51) Int. Cl.: A61B 18/24, A61B 18/26, A61M 25/10, A61B 18/22, A61B 18/00

(54) **LASER SYSTEM FOR DETECTING AND PROCESSING INFORMATION**

(30) Priority: 23.09.2022 EP 22197529
(71) Applicant: Terra Quantum AG, 9000 St. Gallen (CH)
(72) Inventor: SOBOL, Emil N., 9000 St. Gallen (CH); VINOKOUR, Valerii M., 9000 St. Gallen (CH)
(74) Representative: Kretschmann, Dennis

(57) **Abstract**

The present disclosure provides a laser system suitable for a treatment of a cartilage tissue in a joint, comprising: a laser source; a feedback controller, configured to regulate a dosimetry of the laser source to produce spatially and/or temporally modulated laser light; a first optical delivery element, configured to guide the spatially and/or temporally modulated laser light to an area in the joint to irradiate a first part of the area; and a detecting element, configured to detect one or more physical, chemical, mechanical and/or structural characteristics in the area in a real-time; wherein the feedback controller is configured to regulate in a real-time the dosimetry of the laser source based on the real-time detected information pertaining to the one or more physical, chemical, mechanical and/or structural characteristics in the area for a controlled activation of a stem cell outside of the first part of the area to form a hyaline cartilage tissue.

## Description

### Field of the Disclosure

The present disclosure is in the field of a medical laser technology. In particular, the disclosure relates to a laser system that maybe applied for curing human and animal joint tissues diseases, including osteoarthritic cartilage, bones, and ligaments.

### Background

Osteoarthritis (OA) is a degenerative joint disease which is the world's leading cause of pain and disability, affecting nearly 27 million people in the United States alone. Osteoarthritis can cause severe pain and limit motion capability. Osteoarthritis implies the progressive loss of the normal structure and functioning of the articular cartilage, the smooth tissue covering the ends of the moving bones. Degeneration of the cartilage is an irreversible phenomenon, it is incurable, costly, and resists against all known treatments. The widespread prevalence of the OA made the repair and regeneration of the articular cartilage a dominant area of medical research. An analysis by the U.S. Centers for Disease Control and Prevention indicated that in 2003 the total damage due to OA and other rheumatic conditions in the United States constituted approximately $128 billion ($80.8 billion in direct and $47.0 billion in indirect costs), equivalent to 1.2% of the 2003 U.S. gross domestic product. Total costs attributable to the OA have increased substantially since 1997, and that increase is expected to continue because of the aging of the population and the increases in obesity and physical inactivity.

Currently, the standard surgical intervention for the end-stage degenerative joint pathology is the total joint replacement. Early surgical intervention for symptomatic cartilage lesions, including osteotomy and autologous osteochondral graft transplantation, has been suggested to restore normal joint congruity and minimize further joint deterioration. However, these techniques are often not offering long-term clinical solutions, prompting the development of the regenerative medicine and tissue engineering approaches to restore articular cartilage. Other strategies include cell-based (with or without scaffolds) or whole-tissue transplantation techniques. Although current surgical therapeutic procedures of the cartilage repair are clinically useful, they cannot restore a normal articular surface and, in many cases, result in the growth of inferior quality fibrocartilage. The restoration of hyaline type cartilage is still an unsolved problem.

Since osteoarthritis affects various parts or tissues of the joint such as cartilage plate, ligaments, meniscus, periosteum, bone, facet joints, etc., it is extremely difficult to cure multiple joint problems. Other challenges which the known methos are facing include: incomplete repair on each surgery, a long healing time, the need for additional research to optimize the dosimetry for different types of joints, specific combination of OA diseases, or different patients. Since the known methods have limited healing effects during each surgery, more surgery sessions or a surgery session with multiples steps are usually required.

It is well-known that a cartilage is a tissue with the poor self-repair ability due to (1) a small number of cells (chondrocytes) that can form new cartilage matrix; (2) cartilage being an avascular tissue, which means cell nutrition is transported through diffusion of water through natural existing micropores. These natural micropores become blocked with age and disease, and cells without nutrition become inactivated or die.

The most common pathological feature of the post- traumatic OA is a lesion of the articular cartilage plate. If these lesions are relatively large (more than 4 mm in size) and superficial (partial- thickness defects that do not reach the bone), they never repair without external intervention. The lesions up to 1 cm in size can be treated with known laser treatment methods. However, the laser systems according to the prior art, especially the ones implemented in an endoscope with needles to penetrate through the tissues to deliver the laser light to the defective area, only have a localized small area of effect and are incapable of treating intermediate or large defects.

Another problem related to the conventional laser treatment is the overheating of tissues during laser irradiation, which causes tissue denaturation, death of most cells, and inflammation. These result in a formation of fibrous tissues and the necessity of using antibiotics.

On the other hand, for large lesions, implants impregnated with stem cells are often used. One of the problems with large implants is the load mismatch stress at the interface between the implant and the natural cartilage. This inappropriate stress leads to poor or prolonged implant engraftment.

Stem cells are also used for a treatment of the joints. However, one of the biggest problems that arises under such a treatment is that the stem cell implantation often results in the formation of a rough fibrocartilage having inadequate and inappropriate mechanical properties.

The prior art and knowledge are summarized in the following patents and publications:
- US 10 913 943 B2 discloses an application of a pulsed laser source to activate stem cells.
- EP 1 665 997 B1 discloses a method for generating a spatially and temporally modulated laser light.
- Sobol, Emil N., et al. "Laser-induced regeneration of cartilage." Journal of Biomedical Optics 16.8 (2011): 080902, discloses multiple mechanisms in a laser-induced regeneration of cartilage in a joint.
- Sobol, Emil N., et al. "Laser-induced micropore formation and modification of cartilage structure in osteoarthritis healing." Journal of Biomedical Optics 22.9 (2017): 091515 discloses a laser-induced micropore structure formation on the defective cartilage tissue.

### Summary of the Disclosure

The objective of the present disclosure is to provide a device and a method that solve one or more of the above-mentioned problems of the prior art. The present disclosure is defined by the appended claims.

A first aspect of the disclosure provides a laser system suitable for a treatment of a cartilage tissue in a joint, comprising: a laser source; a feedback controller, configured to regulate a dosimetry of the laser source to produce spatially and/or temporally modulated laser light; a first optical delivery element, configured to guide the spatially and/or temporally modulated laser light to an area in the joint to irradiate a first part of the area; and a detecting element, configured to detect one or more physical, chemical, mechanical and/or structural characteristics in the area in a real-time, wherein the feedback controller is configured to regulate in a real-time the dosimetry of the laser source based on the real-time detected information pertaining to the one or more physical, chemical, mechanical and/or structural characteristics in the area for a controlled activation of a first stem cell outside of the first part of the area to form a hyaline cartilage tissue.

In the context of the first aspect, the cartilage tissue may be a cartilage tissue or a cartilage tissue part that requires treatment, namely a cartilage tissue lesion. The cartilage tissue lesion may show a reduced functionality compared with healthy cartilage tissue or compared with a newly formed hyaline cartilage tissue. The cartilage tissue lesion may also refer to the cartilage tissues or cartilage tissue parts whose functionality is not reduced but requires modification to improve a treatment quality.

A seminal idea that the disclosure states is that the maximum healing effect in the laser treatment or in each laser treatment session may be achieved via activating the remote first stem cell into a directed differentiation to form a hyaline cartilage tissue by the localized temporally and spatially modulated laser light.

In some examples, the localized laser light may be absorbed in a tissue region of the volume between 0.01 to 10 mm³, in particular, 0.1 to 1 mm³. A small irradiated area may reduce damage to the tissue due to direct laser irradiation and facilitate a more energy efficient and controllable modulation of the laser light. Although the directly irradiated area according to the present disclosure may be small in some examples, a large area can be treated by the laser-induced effects.

A stem cell may be activated for a directed differentiation through two mechanisms: a) putting the stem cell under a specific stress, electrical and/or temperature conditions, and b) introducing into the stem cell a specific signaling molecule. Therefore, a remote control over the first stem cell can be realized in at least two ways:
(1) A controlled direct thermal-mechanical activation of the first stem cell through a stress wave (a wave due to an oscillating thermal mechanical property of a medium) arising due to a non-uniform heating waves arising, in turn, as a result of the laser-induced coordinated rotational oscillations of water electric dipoles in a cartilaginous matrix. The stress wave may propagate to the first stem cell and activate the first stem cell through generating a specific thermal and/or mechanical condition for the first stem cell. The stress wave may also drive an electric charge, for example distributed on a surface of a gas bubble to the first stem cell to realize the desired electric condition for a directed activation. A controlled gas bubble generation may be induced by the modulated laser light. The gas bubble may also facilitate a generation of a stress wave.
(2) A remote control over the first stem cell as an indirect biochemical activation of the first stem cell by signaling molecules generated by a second cell in the first part of the area. The signaling molecule generation can be induced by the modulated laser light by generating a specific thermal and/or mechanical condition for the second cell. The second cell can be an implanted stem cell or a cell preexisting in the area. Examples of such signaling molecules may comprise TGF-Bs, BMPs, IGFs, FGFs, SOXs and one of a family of molecular chaperones (Hsp 60, Hsp 70 and Hsp 90). The signaling molecules may be prepared separately and not in-vivo. For example, the second cell may be irradiated in-vitro and then injected to the area of the joint. In this case the laser light need not be used to activate the second cell in-vivo, but to treat the area to solely promote the signaling molecules transportation. Both activation schemes may be realized though a real-time modulated laser light based on the real-time detected information pertaining to one or more characteristics in the area. In particular, the one or more characteristics may comprise a physical, chemical, mechanical and/or structural characteristic in the area.

A medical doctor or a medical practitioner may introduce the first optical delivery element to the vicinity of the area manually. A doctor or practitioner may then select a function of the laser system, for example to determine whether to perform a direct thermal-mechanical activation or an indirect biochemical activation and start the laser treatment. After the laser treatment is started, it can run automatically until a detected information reaches a predetermined threshold, for example, when the stress or temperature of the area reaches a predetermined value. When such a threshold is reached, the laser system can either stop the laser treatment or pause and wait for the next command of a doctor or practitioner.

In the context of the present disclosure, "a real-time" may generally refer to a time scale in which the one or more physical, chemical, mechanical and/or structural characteristics of the area in the joint are detected and are subsequently processed, wherein the time scale is sufficiently short to allow the dosimetry of the laser source to be purposefully regulated via the feedback based on the detected and processed information during the ongoing treatment of the cartilage tissue, in particular, during the ongoing controlled activation of the stem cell outside of the first part of the area to form the hyaline cartilage tissue.

In the context of the present disclosure, "a real-time" may refer to a time scale smaller than several minutes. For example, detecting in a real-time may refer to detecting continuously over a time period of several minutes or detecting several minutes after an external effect for evaluation of such an effect. Processing in a real-time may refer to a processing where the result can be calculated several minutes after the calculation starts. Nonetheless, a smaller time scale is likewise possible.

In particular, a real-time may refer to a time scale smaller than 20 minutes, in particular smaller than 10 minutes or smaller than 3 minutes or smaller than 1 minute or smaller than 30 seconds or smaller than 10 seconds or smaller than 1 second.

In the context of the present disclosure, "regulating a dosimetry of a laser" or "regulating a laser" may refer to regulating the laser in operation. However, they may also comprise selecting a proper initial parameter of the laser for starting the laser treatment. In that case, detecting in a real-time may refer to the situation where the characteristics are detected within a time span of up to several minutes before the laser starts to work. The laser may start in different initial conditions depending on the exact situation of the to-be-treated joint.

In an implementation of the laser system of the first aspect, the laser may be regulated by adjusting at least one of the following laser parameters: a laser pulse repetition rate; a pulse sequence frequency; a duration of a laser pulse; a shape of a laser signal in the time domain; a shape of the laser signal in the frequency domain; a laser wavelength; a pulse energy; an intensity of the laser signal; a number of pulses in a pulse series; an interval duration between series; a number of total series; a spatial distribution of laser irradiation intensity; a dimension of irradiated area; a distance between neighboring irradiated areas; and a distance shift due to the propagation in the first optical delivery element.

Adjusting one or more of these parameters may facilitate a fine tuning based on the environment properties, which may increase the precision of the laser light modulation and the range of applications.

A real-time regulation of the dosimetry of the laser source may correspond to a constant adjustment of a laser dosimetry, adjusting the laser dosimetry upon receiving a signal from the feedback controller or updating the laser dosimetry after a certain number of pulses in a sequence. A real-time regulating may further comprise stopping the irradiation when the real-time detected information pertaining to the characteristic in the area reaches a predetermined or calculated threshold.

In a further implementation of the laser system of the first aspect, the detecting element may comprise at least one of the following: an X-Ray device; a CT device; an ultrasonography device (US); an MRI device; an OCT device; a (multispectral) optoacoustic tomography device (MSOT); a fluorescence molecular tomography device (FMT); and an acoustic tomography device.

These types of detecting elements may provide high resolution monitoring of the area or a local environment but may generate a large volume of data. Through combining a plurality of different types of the detecting elements with a powerful (built-in or external) computer, for example a quantum computer, the disclosure may facilitate a precise real-time control of the laser regulation.

In a further implementation of the laser system of the first aspect, the characteristics may comprise one or more of the following: a Young's modulus of an object, a speed of sound in an object, a temperature of an object and/or an environment, a position of an object, a composition of an object, a dimension of a lesion, a thickness of the cartilage plate, plurality and dimensions of structural defects, a shape of the implant, a dimension of the collagen fibrils, a type of the collagen in the object, amount of proteoglycan in cartilage, a stress distribution on an object, a light scattering induced by an object, a conductivity of an object, a characteristic pertaining to a porous structure and/or a zone of denaturized tissue on the object such as a porosity of the porous structure.

The characteristics maybe of one or more objects inside the first part of the area to reflect a primary effect of the laser light and/or for a calculation of a desired generation conditions of the stress wave induced by the laser light, or for a calculation of a desired condition to stimulate the second cell to release the signaling molecule. These characteristics may be of one or more objects outside the first part of the area to reflect a secondary effect of the laser light and/or for a calculation of a desired propagation of the stress wave induced by the laser light or a desired transportation of the signaling molecules. The characteristics maybe of one or more objects where the remote first stem cell is located to reflect and/or for a calculation of the final effect of the laser light induced remote controlling effect on the remote stem cell.

In an example, due to the complexity of both the direct thermal-mechanical activation and the indirect biochemical activation schemes, as well as the complicated environment in the human body, the desired result may be achieved through regulating multiple dosimetry parameters of the laser source at the same time based on a calculation of the real-time detected information pertaining to multiple characteristics of the area through a specially designed algorithm.

Due to the large number of input parameters and output parameters of the algorithm, a large feedback lag may lead to a divergence from the desired effect. Real-time control may be achieved by high-performance computing power. In an example, such a computer can be a (remote) high-performance computer, a (remote) hybrid quantum-classical computational facility, and/or a (remote) quantum computer.

In a further implementation of the laser system of the first aspect, the feedback controller may be configured to regulate the dosimetry of the laser source, so that the generated modulated laser light changes a temperature and/or a stress of a tissue in the first part of the area in a specific sequence and/or simultaneously.

In a further implementation of the laser system of the first aspect, the feedback controller may be configured to regulate the dosimetry of the laser source, so that the generated modulated laser light changes a temperature and/or a stress field, in particular a temperature and/or stress distribution in the first part of the area in a specific sequence and/or simultaneously.

In a direct thermal-mechanical activation, changing a temperature and/or a stress of a tissue in the first part of the area in a specific sequence can facilitate a generation of a desired stress wave. The stress wave can also be a superposition of different thermal-mechanical waves, so that the temperature and /or the stress of the tissue can be changed simultaneously. In an indirect biochemical activation, modifying the temperature and the stress of the tissue in the first part of the area in a specific sequence and/or simultaneously facilitates creating a desired temperature and stress condition to stimulate a stem cell in the first part of the area to generate signaling molecules. Changing a temperature and/or a stress of a tissue in the first part of the area in a specific sequence and/or simultaneously can also facilitate a controlled formation of porous structure.

In a further implementation of the laser system of the first aspect, the system may comprise: a channel element configured to create an access channel to the area in the joint.

In a further implementation of the laser system, the channel element is configured to deliver the to be activated stem cell to the area.

In a further implementation of the laser system, the channel element is configured to deliver a pre-activated stem cell to the area.

In a further implementation of the laser system, the first optical delivery element is configured to guide the spatially and/or temporally modulated laser light through the channel element to the first part of the area.

The to be activated stem cells can be introduced from an external source. These stem cell can be introduced to a desired location of the osteoarthritis joint for a cartilage tissue reparation. The channel element may comprise a same lumen for the stem cell introduction and laser light guiding. For example, the first optical delivery element may be inserted through the lumen before or after the stem cell is introduced through the lumen. Using the same channel element, especially through the same lumen for stem cell introduction and laser light guiding minimizes the trauma due to the access channel formation.

In an example, the quantity, density and/or other dosimetry of the stem cell (for example dispersed in a biological fluid) may be controlled by the feedback controller in a real-time based on the real-time detected information.

In a further implementation of the laser system of the first aspect, the feedback controller may be configured to control in a real-time, based on the real-time detected information, a position of the first optical delivery element in the area during the irradiation.

For example, the feedback controller may be configured to control the first optical delivery element to guide the laser light to a second part of the area different from the first part of the area in a real-time based on the real-time detected information pertaining to the one or more physical, chemical, mechanical and/or structural characteristics in the area.

The first optical delivery element may comprise a single or a bundle of optic fibers. The first optical delivery element may comprise a plurality of outcoupling elements. The change of the irradiated part of the area may comprise a switching between different outcoupling elements and/or controlling individual outcoupling elements, such as tilting an angle of the outcoupling element. The first optical delivery element may comprise a servo element, configured to change the physical location of the optical delivery element for changing the irradiated part of the area. The irradiated part of the area may be changed after an activation of the remote stem cell, to activate another remote stem cell. The changing of the irradiated part of the area may also be performed while activating the same remote stem cell. In the latter case, the change of the irradiated part of the area, as a part of the spatial modulation of the laser light, may facilitate a generation of a desired stress wave or a generation of a desired signaling molecule.

In a further implementation of the system of the first aspect, the feedback controller may be configured to regulate the dosimetry of the laser source for a controlled formation of a porous structure on the cartilage tissue and/or another object in the area based on the real-time detected information.

In the context of the present disclosure, a porous structure may refer to a structure with a distributed plurality of structural defects. A pore in such a porous structure does not need to be of a rounded shape in a conventional sense, but can also be a creek, microcavity, displacement, or another form of a structural defect that promote transfer of liquids and signal molecules through the tissue matrix. Yet a porous structure should also be distinguished from an assembly of macrofractures, that impairs the mechanical properties of the tissue. In an exemplary configuration, the porous structure may be a microporous structure, that is to say, the structural defects can have the size less than 5 micrometers. At the initial stage of formation of such a microporous structure, the formation may only modify the physical and chemical properties of the affected area and may not significantly change the macroscopic appearance and mechanical properties of the tissues.

A microporous structure can be formed on the cartilage tissue in the area in a controlled manner. In one example, this microporous structure is formed prior to the laser-induced activation of the remote stem cell.

In some examples, the tissue and/or the object on which the porous structure is formed may already be porous before the laser-induced porous formation. In these examples, a porous structure refers to a structure with an increased porosity over the untreated tissue or object. In some examples, a porous structure formation may refer to an increase in the porosity and/or an increase in the pore sizes. This makes it possible to control the mechanical properties of the tissues and the objects, in particular their tensile strength, conditions defining stress waves propagation and other elastic and plastic properties. In another example, a porous structure formation may refer to a de-clogging of a preexisting clogged porous structure. Since clogging may often occur in an unstable porous structure, the controlled porous structure formation according to the present disclosure may be applied to de-clog the clogged unstable porous structure and form a stabilized porous structure for a long-time stability.

For a direct thermal-mechanical activation, the stress wave can propagate through the cartilage tissue from the irradiated part of the area in the joint to the stem cell on a surface of the cartilage tissue outside the irradiated first part of the area. Forming a porous structure in a controlled manner may improve the stress waves generation and the stress waves propagation. On one hand, the porous structure formation may change the porosity of the tissue, therefore changing, and, in particular, increasing, the interaction between the modulated laser light and the tissue due to the increased liquid content in the porous structure, facilitating the generation of the stress waves. On the other hand, a porous structure can change the physical properties of the cartilage tissue as the medium harboring the stress waves propagation, in particular the speed of sound and the Young's modulus. The cartilage tissue can therefore be engineered in this way to optimize the stress wave propagation.

In an indirect biochemical activation, the porous structure can provide an extra transport pathways, namely through the pores, for the signaling molecules and can, therefore, promote the propagation of the signaling molecules to the remote stem cell. In both cases, it can be beneficial to use the controlled porous structure formation to promote the activation of the stem cells attached to the cartilage tissue to form a cross-linkage of hyaline cartilage tissue on the surface thereof, hence facilitating an improved healing effect. In an example, the size of the pores, or the porosity of the porous structure, can be large enough to optimize the stress waves propagation, or to promote the signaling molecules transportation. In another example, the size of the pores can be small enough to impede the moving of the stem cells through the porous structure. In this way, the stem cells can be effectively distributed over the damaged cartilage tissue hindering an aggregation of the stem cell, therefore enlarging the area of the healing effect. In another example, the size of the pores is controlled within a certain range to maximize their stability.

The porous structure may reduce an internal stress of an object, for example the to-be treated tissue. In the present disclosure, an internal stress may refer to a permanent or static stress in an object, which is different from the dynamic oscillating stress change in a stress wave. The internal stress may occur or be detected throughout the treatment, including scaffold implantation or laser irradiation, which may be an undesired side effect. Detecting the internal stress and reducing it through forming a porous structure may improve the quality of the laser treatment and/or scaffold implantation. Therefore, the porous structure may be implemented at any stage of the laser treatment to reduce an internal stress in the area in the joint.

In addition, the porous structure may further promote a transportation of a drug and nutrition in the cartilage tissue area and improve the treatment effect. In an example, the channel element may be configured to deliver the drug, nutrition and/or other biochemical substances before, during and/or after the irradiation.

The porous structure may further be formed in another object in the area in the joint, for example, in an implant. Through doing this, the conditions for different biochemical substances to be easily transported through the implant or other objects in the area in the joint are created.

As outlined above, the laser light may be only used to generate the porous structure and not activate the second cell. In this example, the signaling molecules maybe prepared separately and introduced to the area after the porous structure has been formed. In this way, the activation of the second cell can be spared, therefore dealing less damage to the injected stem cells.

In a further implementation of the laser system of the first aspect, the detecting element may comprise an optical receiving element.

In a further implementation of the laser system of the first aspect, the optical receiving element may be configured to receive a scattered light.

The scattered light may stem from the spatially and/or temporally modulated laser light. Light scattering may be sensitive to the formation of microporous structures, or other defects on a microscopic level. Detecting and analyzing the scattered light of different wavelengths makes it possible to use the Mie and Rayleigh scattering laws, to determine the size distributions of pores, defects, or potential gas bubbles in the area in the joint, which may be generated during the porous structure formation, or stress wave generation.

In a further implementation of the laser system of the first aspect, the detecting element may comprise a second optical delivery element.

In a further implementation of the laser system of the first aspect, the optical delivery element may be configured to deliver a probing light signal for light scattering analysis.

Scattered light may also stem from a probing light signal. The probing light signal needs not to interact with the tissue or the object so as to form pores or tissue denaturation and can be used before the laser operation to determine the initial condition of the laser. This may facilitate an initial condition of the laser with little or no destructive side effect on the tissue or the object or their environment.

In a further implementation of the laser system of the first aspect, the first optical delivery element comprises a bundle of optical fibers and/or is configured to multiplex a plurality of the laser outputs of the laser source into one fiber at an input of the first optical delivery element.

This may improve a flexibility of the spatial modulation of the laser light as well as the scattered light detection mentioned above.

In a further implementation of the laser system of the first aspect, the detecting element may comprise a conductivity detecting element.

In a further implementation of the laser system of the first aspect, the conductivity detecting element may be configured to detect a conductivity on the tissue or on the object.

In some examples, it may be beneficial to form a stabilized porous structure. This can be realized through a stabilized gas bubbles generation. A spatial and/or temporal modulated laser light is capable of generating the microbubbles from the gas dissolved in a liquid in the environment. These bubbles may be stabilized by positive charges at their surfaces. The conductivity information may, therefore, reflect the status of the bubble formation. Modulating the laser light considering this information may facilitate a controlled generation of stabilized gas bubbles. It may further facilitate a controlled formation of the stabilized porous structure.

As outlined above, the porous structure can have multiple different functions in the laser treatment. The characteristics of the porous structure, for example the pore width and length can be important parameters in optimizing other process, such as stress wave generation, stress wave propagation, signaling molecules transportation and/or transportation of other biochemical substances. Therefore, the controlled formation and an accurate detection of the corresponding characteristics can be important to achieve the effect of the laser treatment. In particular, a real-time feedback controller may realize such controlled formation and/or real-time detection of pores.

In a further implementation of the system of the first aspect, the first optical delivery element may be configured to irradiate the first part of the area to induce a formation of a cross-linkage of hyaline cartilage tissue between an implant and a cartilage tissue in the area.

The cartilage tissue may be or comprise a cartilage tissue in a lesion area, in particular, adjacent to or in the vicinity of the implant before, during or after the cross-linkage formation, which may be considered to effectively form a cartilage tissue lesion.

In such an implementation, the implant maybe delivered to the cartilage tissue through the channel element.

If the lesion area in the joint is large enough, it may be beneficial to introduce an implant to the lesion area and form a cross-linkage of hyaline cartilage tissue through activating the stem cells to a directed differentiation between the cartilage tissue and the implant.

The interface between the implant and the cartilage tissue may be larger than a laser spot diameter and may require multiple irradiation steps to establish a stable cross-linkage between the cartilage tissue and the implant according to a conventional laser treatment. According to the present disclosure, one localized irradiation may induce the cross-linkage formation throughout a large part of the interface between the implant and the cartilage tissue. Therefore, much less irradiation steps are required. The efficiency may be increased, and the surgical trauma may be reduced.

In a further implementation of the system of the first aspect, the detecting element may be configured to detect a stress or a stress distribution at or in the vicinity of the interface between the implant and the cartilage tissue.

In a further implementation of the system of the first aspect, the stress or stress distribution at or in the vicinity of the interface between the implant and the cartilage tissue may be detected prior, during and/or after the cross-linkage formation.

In a further implementation of the system of the first aspect, the feedback controller may be configured to control the laser source and the first optical delivery element to form the porous structure at or in the vicinity of the interface between the implant and the cartilage tissue according to the detected stress or stress distribution.

In a further implementation of the system of the first aspect, the feedback controller may be configured to control the laser source and the first optical delivery element to reduce stress due to the formation of the porous structure at or in the vicinity of the interface between the implant and the cartilage tissue according to the detected stress or stress distribution.

After the cross-linkage is formed between the implant and the cartilage tissue, a mismatch stress may occur at or in the vicinity of the interface between the implant and the cartilage tissue. A laser-induced porous structure may reduce an internal mechanical stress of a rigid object. However, an uncontrolled formation of the porous structure may destroy the freshly formed cross-linkage of the hyaline cartilage tissue. Therefore, the porous structure, preferably a microporous structure should be formed in a controlled manner. This can be realized utilizing a real-time laser regulation scheme according to the present disclosure. The above-mentioned system suitable for forming a porous structure on the cartilage tissue prior to the stem cell activation may be directly used in this example. The controlled formation of porous structure based on the real-time detected information in these examples may be implemented using the same method.

In a further implementation of the laser system of the first aspect, the laser system may comprise an effect exerting element, configured to exert a thermal, electrical, magnetic and/or mechanical effect in the area, wherein the feedback controller is configured to regulate the effect exerting element in a real-time based on the real-time detected information.

In a further implementation of the laser system of the first aspect, the effect exerting element may exert the thermal, electrical, magnetic and/or mechanical effect on a tissue, an object, and/or a fluid in the area.

The tissue may be or comprise the cartilage tissue and/or a different tissue. In particular, the tissue may be outside the illuminated part of the area.

An extra effect exerting element is configured to exert another chemical, thermal, electrical, magnetic, acoustic and/or mechanical effect in addition to or during the laser irradiation in the area to increase the quality of the remote communication effect.

For example, the effect exerting element may comprise a heater. The system with the heater may facilitate a better temperature control, especially when the laser intensity is low in some cases.

As another example, the effect exerting element may comprise an electrical effect exerting element. The porous structure can be stabilized by a charge distributed at surfaces of the gas bubbles. Although it is possible to use laser effect alone to generate such charge locally, an electrical effect exerting element may introduce external charges to the area in the joint to improve the stability of the gas bubbles. As another example, the electrical effect exerting element may be configured to create an electrical field in the area in the joint. As another example, the effect exerting element may exert a piezoelectric effect in the area in the joint, for example to exploit a collagen piezoelectric property.

As another example, the effect exerting element may comprise a mechanical oscillator. The mechanical oscillator may be regulated by the feedback controller in a real-time together with the laser source to produce a stress wave to activate the first stem cell. The mechanical oscillator may be configured to generate an acoustic wave such as an ultrasonic wave. Through combining the mechanical and the optical effects, the stress wave can be optimized, and the damage done during the stress wave generation may be reduced. As another example, the mechanical oscillator may physically contact a to be treated cartilage tissue. As another example, the effect exerting element may comprise or be the same as the servo element. As another example, the effect exerting element may comprise or be the same as a guide wire.

In some examples, the feedback controller may utilize the combination of one or more effect exerting elements as well as the laser source as a whole and regulate the effect exerting elements as well as the laser source at the same time to achieve an optimal effect.

In a further implementation of the system of the first aspect, the feedback controller may be configured to regulate the laser source and/or the effect exerting element to activate or deactivate a nerve ending in a real-time base on the real-time detected information.

Signaling molecules (neurotransmitters) may be synthetized in and released from nerve endings and then bind to receptor proteins in the cellular membrane of the target tissue. The target tissue may get excited, inhibited, or functionally modified in some other way.

For a better control of the healing effect, it may be beneficial to activate or deactivate a nerve ending on demand, for example, to decrease the generation of unwanted signaling molecules from the body or to promote a generation of desired signaling molecules from the body. A possible deactivation or activation of nerve ending may comprise exposing the nerve ending to one or more of the thermal, mechanical, electrical, and optical effects, for example, the ones similar to the examples outlined above, so that a desired temperature requirement, local stress requirement, local charge requirement or other requirements of a deactivation or activation of nerve ending can be achieved.

A possible activation may further comprise a removal or a decrease of a size of an object compressing the nerve. For example, a low-temperature laser ablation method may be performed to reduce a fibrous cartilage tissue compressing the nerve: the feedback controller may regulate the laser source to form a porous structure on the fibrous cartilage tissue in a controlled manner. A biological fluid, such as synovial fluid in the area may then flow into the pores. The increased biological fluid content in the porous structure may increase an interaction between the porous structure and the laser light. Therefore, a thermal-mechanical gradient may occur between the area of the porous structure and the neighboring area which is not affected by the laser. This may lead to an ablation of the irradiated fibrous cartilage tissue. The laser dosimetry, especially a time interval between the pulses may be regulated in a real-time, so that, for example, the time interval is long enough for the biological fluid to fill the porous structure, and/or short enough to guarantee an ablation safety.

In a further implementation of the system of the first aspect, the feedback controller may regulate the dosimetry of the laser source, the dosimetry of the effect exerting element and/or the dosimetry of the introduction of the biochemical substance in a real-time based on a combination of the real-time detected information, the dosimetry of the laser source, the dosimetry of the effect exerting element and/or the dosimetry of the introduction of biochemical substance.

The output of the feedback controller regulating a part of the laser system may also be used as a feedback information to regulate the same or a different part of the laser system. In some examples, different biochemical substances introduced into the area in the joint may change the tissue properties in this area in the joint, especially the optical and mechanical properties. For example, the feedback controller may determine a volume of the biochemical substance to be introduced in the area of the joint. The controller may then determine that an elasticity in this area may be increased after the introduction of the biochemical substance, and a stress wave propagation or signaling molecule transportation may become quicker. The feedback controller can then regulate the corresponding parts of the laser system accordingly.

In a further implementation of the system of the first aspect, the feedback controller may comprise and/or be coupled to a (remote) high-performance computer, a (remote) hybrid quantum-classical computational facility, and/or a (remote) quantum computer.

In a further implementation of the system of the first aspect, the feedback controller may comprise and/or be connected to a storage device, the storage device storing an offline settings table, wherein the settings table is calculated by a remote high-performance computer, a remote hybrid quantum-classical computational facility, and/or a remote quantum computer.

The real-time regulation of the laser based on feedback detected information of the laser affected area according to the present disclosure is a complex feedback optimization problem. A better evaluation of the laser effect and a precise regulation of the laser relies on a large volume of the detected information, which may be an information of a huge volume. Quantum algorithms or hybrid-quantum algorithms such as a variational quantum eigensolvers may be employed in this context and may outperform a conventional algorithm in optimizing a system with parameters of multiple dimensions. Therefore, using a high-performance, and /or hybrid-, and/or quantum-computer, and/or a hybrid computational facility may facilitate a better control over the laser system.

In a further implementation of the method of the first aspect, the remote high-performance computer, the remote hybrid quantum-classical computational facility, and/or the remote quantum computer may be located in a central server.

In a further implementation of the method of the first aspect, the central server is configured to regulate a plurality of laser systems.

A second aspect of the disclosure provides a method for detecting and processing information, comprising:
a) detecting one or more physical, chemical, mechanical, and/or structural characteristics in an area in a joint;
b) processing the detected information pertaining to the physical, chemical, mechanical and/or structural characteristics in the area in the joint; and
c) acquiring a property of a stress wave generation, a stress wave propagation, a signaling molecule generation, a signaling molecule transportation, and/or a porous structure formation in the area in the joint.

In an implementation of the method of the second aspect, the one or more physical, chemical, mechanical, and/or structural characteristics in the area maybe one or more physical, chemical, mechanical, and/or structural characteristics of a tissue, an object, and/or a fluid in the area.

The tissue may be or comprise the cartilage tissue and/or a different tissue.

In a further implementation of the method of the second aspect, the method may further comprise the following step:
d) promoting an interaction between an object in an irradiated part of the area and an object outside the irradiated part of the area.

In a further implementation of the method of the second aspect, the information detected and processed may be used for the promoting of the interaction.

In a further implementation of the method of the second aspect, the object in the irradiated part of the area may be a fluid, tissue, implant, or a cell, such as a normal cell or a stem cell.

In a further implementation of the method of the second aspect, the object outside the irradiated part of the area may be a cell such as a normal cell or a stem cell.

In a further implementation of the method of the second aspect, the interaction may comprise or may be a thermal, mechanical and/or electrical interaction propagated via a stress wave.

In a further implementation of the method of the second aspect, the interaction may comprise or may be an exchange of information between a cell in an irradiated part of the area and a cell outside the irradiated part of the area, for example molecular signalling information exchanged via a signalling molecule.

In a further implementation of the method of the second aspect, the promoting of the interaction may comprise a direct promoting, for example by optimizing the stress wave generation, the stress wave propagation, the signalling molecule generation and/or the signalling molecule transmission.

In some embodiments, the promoting of the interaction may comprise an indirect promoting, for example by optimizing the formation of a porous structure, which indirectly promotes the stress wave propagation and/or signalling molecule transmission.

In a further implementation of the method of the second aspect, the exchange of information may refer to interacting directly with a first cell and use the first cell to interact with the second cell indirectly. The first cell may be located in the irradiated part of the area. The second cell may be outside the irradiated part of the area.

In a further implementation of the method of the second aspect, the information detected and processed is used to regulate a laser light in a real-time for promoting the exchange of information between the cell in the irradiated part of the area and the cell outside the irradiated part of the area, wherein the laser light is used to irradiate the part of the area.

In a further implementation of the method of the second aspect, the cell in the irradiated part of the area and/or the cell outside the irradiated part of the area can be a stem cell.

In a further implementation of the method of the second aspect, the method may further comprise: promoting the signalling molecules emitted by the laser-activated stem cells to activate other remote cells to differentiate or dedifferentiate in a desired direction.

In a further implementation of the method of the second aspect, the stress wave and/or the signaling molecule may be configured to activate a stem cell to form a hyaline cartilage tissue. In a further implementation of the method of the second aspect, the acquiring of the property of the stress wave generation, the stress wave propagation, the signaling molecule generation, the signaling molecule transportation, and/or the porous structure formation in the area may be performed in a real-time during the stress wave generation, the signaling molecule generation, and/or the porous structure formation.

The area may comprise a solid medium, a liquid medium or a combination of them for a stress wave propagation and signaling molecule transportation. For example, the medium for a stress wave propagation and signaling molecule transportation may be a defective tissue or an implant or other object. It may also be a biologic fluid in the treatment environment. It may further refer to a tissue or an implant comprising a porous structure filled with the biologic fluid.

A property of a stress wave generation may comprise a time dependent temperature and/or stress change in the area where the stress wave is generated.

A property of a stress wave propagation may comprise a time dependent temperature and/or stress change in the area where the stress wave propagates. The property of stress wave propagation may further comprise a Young's modulus or a speed of sound of the medium.

A property of signaling molecule generation may comprise a temperature range and/or a stress range promoting the second cell to generate the signaling molecules.

A property of signaling molecule transportation may be related to the porous structure, for example, to a pore shape and size, a channel length in the porous structure, or other property reflecting the signaling molecule transportation in the porous structure. A property of signaling molecule transportation may further comprise the properties related to a biological fluid assisted drift or diffusion. For example, the property may comprise a thermal-mechanical gradient indicating the drift of the signaling molecule assisted by the fluid flow. This property may also comprise a temperature in the area in a joint promoting the diffusion of the signaling molecule in the biological fluid.

A property of the porous structure formation may comprise a pore size, a stability, a quality, and/or a property used for an evaluation of the porous structure formed, or a formation speed, a stability, a quality, and/or other properties used for evaluation of the porous structure formation procedure.

In a further implementation of the method of the second aspect, the stress wave generation, the stress waves propagation, the signaling molecule generation, the signaling molecule transportation, and/or the porous structure formation may be induced by a temporally and/or spatially modulated laser light generated by a laser source.

Different stress waves generation, stress waves propagation, signaling molecule generation, signaling molecule transportation, and/or porous structure formation mechanisms may require different temperatures. The required temperature may reflect what happens in the area, whether a desirable effect is reached as expected, or whether a potential damage may be caused. Further, the same physical, chemical, mechanical and/or structural characteristics may reflect different properties. For example, a time dependent stress distribution may both reveal the stress wave propagation, and deliver an information on a size distribution of the pores, or indicate the formation of denaturized tissue.

For example, a detected stress distribution may be mapped onto the detected temperature, since it may provide a more precise evaluation of the laser irradiation effect. The combination of the temperature detection and a mechanical stress detection may reflect different properties in the area in the joint and may facilitate a precise evaluation of the laser effect. Other physical, chemical, mechanical and/or structural characteristics in the area in the joint may provide information for a better evaluation of the properties in this area and a laser effect in this area.

Although the present disclosure may provide an automatic feedback-control laser system, for example the laser system according to the first aspect, it is understood that the method according to the second aspect need not encompass the regulation of laser system itself. For example, the method according to the second aspect can provide the doctor or the practitioner operating a laser with the necessary information based on which the doctor or the practitioner may subsequently evaluate the lesion area in the joint and/or an expected laser effect in this area.

An evaluation system, configured to perform the method according to the second aspect may comprise an indicator, for example an indicating LED light bulb. In an example, if the evaluation system determines that the to-be treated tissue in the joint shows a large lesion area, it may indicate to the doctor or the practitioner to perform a laser treatment, for example, through showing a green light. It may further indicate the doctor or the practitioner where to perform a laser treatment, for example through showing the corresponding information on a screen. In another example, if the evaluation system determines that the detected information in the to-be treated joint reaches a predetermined value, for example if a stress is small enough, or a temperature is too high, it may indicate to the doctor or the practitioner to stop the laser treatment, for example through showing a red light. The method may also provide indications to the doctor to perform other actions, for example to change the dosimetry of the laser. The threshold, the predetermined value and/or other evaluation criteria may be predetermined by the doctor, or the practitioner based on concrete cases, or stored in an offline settings table for the treatment, wherein the setting table may be calculated by a remote high-performance computer, a remote hybrid quantum-classical computational facility, and/or a remote quantum computer. The threshold, the predetermined value and/or other evaluation criteria may be predetermined based on the laser used by the doctor or the practitioner, which may be a laser in the laser system according to the first aspect of the disclosure. The laser may also be a laser where the dosimetry can be adjusted manually.

In a further implementation of the method of the second aspect, the processing the detected information may comprise: generating a value for a dosimetry of a laser source in a real-time based on the detected information pertaining to the physical, chemical, mechanical and/or structural characteristics in the area.

In a further implementation of the method of the second aspect, the value may be generated in a real-time during the stress wave generation, the signaling molecule generation and/or the porous structure formation.

In an automatic laser system such as the laser system according to the first aspect of the disclosure, the generated value for a dosimetry of the laser may be directly used by the feedback controller to regulate the laser dosimetry without a human interference. The value may also be displaced to the doctor or the practitioner, so that they can use it to manually adjust a laser dosimetry or to decide whether to stop the laser treatment. As long as the detecting and the processing of the information can be performed in a real-time, for example within several minutes, the doctor or the practitioner may have enough time to react to change the laser dosimetry in time for a real-time laser effect, even if the doctor or the practitioner chooses to change the laser dosimetry manually. Compared to the conventional monitoring and evaluation systems, an evaluation system adopting the method according to the second aspect provides a more informative and precise feedback to the doctor or the practitioner operating a laser system for treating a joint.

In a further implementation of the method of the second aspect, the detecting of the physical, chemical, mechanical and/or structural characteristics in the area may comprise: determining a temperature or a temperature field in the area, wherein the value for the dosimetry of the laser source is generated if the temperature and/or its distribution is within a predetermined range.

In a further implementation of the method of the second aspect, the value for the dosimetry of the laser source may not be generated if the temperature is not within the predetermined range.

In a further implementation of the method of the second aspect, the laser treatment may be terminated if the temperature is not within the predetermined range.

For the laser treatment according to the present disclosure, the parameter may be adjusted so that the detected temperature can be maintained within a specific range defining the activation condition of the stem cell using the stress wave or the signaling molecules. For example, the second cell heated by the modulated laser light can be heated up to 40-45°C for a few seconds, for example, in a time interval less than twenty seconds. This can activate the second cell to generate signaling molecules.

As another example, the temperature where the stress wave is generated can be changed and controlled by the modulated laser light according to the desired reachable range of the stress wave, and a behavior of the stress wave dissipation in the environment.

For a stabilized porous structure formation, the parameters maybe adjusted so that the detected temperature can be maintained below the temperature threshold, within a predetermined range. The gas bubbles can be stabilized in a lower temperature range. In an exemplary configuration, the temperature threshold may be determined to be a value no smaller than 40 °C and/or no larger than 80 °C.

For the deactivation of compressed nerve endings, the parameters may be adjusted in such a way that the detected temperature can be maintained above the temperature threshold from 50°C to 90°C. This may increase the efficiency of the laser effect and may reduce the waste of energy. It may, therefore, facilitate an efficient use of resources.

For example, a monitoring system performing the process of the second aspect may process the temperature according to the current task and/or other characteristics in the area in the joint to provide the practitioner or the doctor the supplementary information on how to operate the laser system.

In a further implementation of the method of the second aspect, the processing of the detected information pertaining to the physical, chemical, mechanical and/or structural characteristics in the area in the joint may further comprise a consideration of the effect of detected temperature on the thermodynamic parameters of the medium, in particular, on the nonlinearity of the thermodynamic parameters.

Laser lights during the laser treatment may be absorbed by the medium. Most modern diagnostic techniques based on a laser treatment ignores the effect of the local temperature increase of the medium on the thermodynamic parameters of the medium (e.g., thermal conductivity, density, thermal-expansion coefficient, and isobaric specific heat capacity) due to such laser light absorption. The conventional diagnostic techniques assume that the thermodynamic parameters are constant. However, even a small increase in local temperature can change the values of thermodynamic parameters of the medium, and it is necessary to consider the nonlinearity of the thermal parameters in the thermal diffusion thermo-mechanical equations. Most serious alterations in the parameters can be due to structural and phase transformation occurring in the tissue during laser irradiation.

In a further implementation of the method of the second aspect, the physical, chemical, mechanical and/or structural characteristics may comprise a characteristic of a scattered light.

In a further implementation of the method of the second aspect, the processing of the detected information may further comprise calculating a pore size distribution of the porous structure based on the scattered light.

In a further implementation of the method of the second aspect, the laser may be configured to generate gas bubbles from gas molecules dissolved in a liquid in the environment.

This may facilitate formation of a stabilized microporous structure, which may change the stress on the tissue or object without destroying it.

In a further implementation of the method of the second aspect, the processing of the detected information may further comprise calculating a stress distribution and/or a temperature distribution in an area.

In a further implementation of the method of the second aspect, the processing of the detected information may comprise: analyzing a thermomechanical gradient in the area.

In a further implementation of the method of the second aspect, the processing of the detected information may comprise mapping the stress distribution to the temperature distribution and/or evaluating the correlation between the stress distribution and the temperature distribution.

A spatially resolved distribution may provide more information about the laser effect, which may increase the precision of the laser regulation.

In a further implementation of the method of the second aspect, the method may comprise acquiring a property of a formation of a cross-linkage of a tissue in a real-time during the cross-linkage formation.

Compared to the laser treatment, the hyaline tissue formation may happen on a much larger time scale. Nonetheless, it maybe possible to process the physical, chemical, mechanical and/or structural characteristics of the tissue in the area in the joint to indirectly infer, whether the condition of a hyaline tissue cross-linkage formation has been reached and/or whether the cross-linkage formation has already started. This may be inferred through a minor change of the physical, chemical, mechanical and/or structural characteristics in the area in the joint compared to the same characteristics prior to the activation.

In a further implementation of the method of the second aspect, the method may comprise detecting a stress distribution at or in the vicinity of an interface between a cartilage tissue and an implant.

The cartilage tissue may be or comprise a cartilage tissue in a lesion area, in particular, adjacent to or in the vicinity of the implant before, during or after the cross-linkage formation, which may be considered to effectively form a cartilage tissue lesion.

In a further implementation of the method of the second aspect, the detected stress distribution may be used for changing the stress at or in the vicinity of an interface between a cartilage tissue and an implant.

In a further implementation of the method of the second aspect, the method may further comprise determining a location at or in the vicinity of the interface between a cartilage tissue and the implant where a stress reaches a predetermined value.

This location may correspond to the area that needs to be treated or the location with a residual stress. For example, laser radiation can be used for generating a porous structure to reduce the stress at this location.

Examining a residual stress at or in the vicinity of the interface between the implant and the cartilage tissue and relaxing it for example through a laser treatment may reduce the chance of apoptosis of the cells in the tissue and promote an implant engrafting. This may increase effectively the per-operation output of the laser radiation and may optimize the use of resources.

In a further implementation of the method of the second aspect, the processing of the detected information may be performed in a (built-in or remote) high-performance computer, a (built-in or remote) hybrid quantum-classical computational facility, and/or a (built-in or remote) quantum computer.

In a further implementation of the method of the second aspect, the method of the second aspect may be encompassed in an algorithm designed for the high-performance computer, the hybrid quantum-classical computational facility, and/or the quantum computer.

In a further implementation of the method of the second aspect, the remote high-performance computer, the remote hybrid quantum-classical computational facility, and/or the remote quantum computer may be located in a central server.

In a further implementation of the method of the second aspect, the central server is configured to regulate a plurality of laser systems.

A third aspect of the disclosure provides a method for treating a joint using a temporal and/or spatial modulated laser light comprising a treatment step, wherein the treatment step comprises:
a) detecting one or more physical, chemical, mechanical and/or structural characteristics in an area in the joint, and feedbacking the detected information pertaining to the physical, chemical, mechanical and/or structural 1 characteristics in a real-time to a feedback controller; and
b) modulating the laser light irradiating a first part of the area in a real-time by the feedback controller based on the real-time detected information, the modulating suitable for an activation of a first stem cell outside the first part of the area to form a hyaline cartilage tissue.

In an implementation of the method of the third aspect, a porous structure may be formed in the tissue and/or in an implant.

In an implementation of the method of the third aspect, the porous structure may be formed with the controlled length and/or width of the pores.

In a further implementation of the method of the third aspect, the first stem cell may be introduced to the area in the joint.

In a further implementation of the method of the third aspect, the porous structure may be formed to diminish a mismatch stress between a scaffold with cells and a native tissue in the area.

In a further implementation of the method of the third aspect, a second cell in the first part of the area in the joint may be activated to generate signaling molecules, wherein the signaling molecules are configured to activate the first stem cell.

In a further implementation of the method of the third aspect, a stress wave is induced through the modulated laser light to activate the first stem cell.

In a further implementation of the method of the third aspect, wherein the method is performed in a (built-in or remote) high-performance computer, a (built-in or remote) hybrid quantum-classical computational facility, and/or a (built-in or remote) quantum computer.

### BRIEF DESCRIPTION OF THE DRAWINGS

To illustrate the technical features of embodiments of the present disclosure more clearly, the accompanying drawings describing the embodiments are introduced briefly in the following description. The accompanying drawings in the following description are merely some embodiments of the present disclosure, and modifications of these embodiments are possible without departing from the scope of the present disclosure as defined in the claims.
- FIG. 1: is a schematic illustration of a laser system according to an embodiment,
- FIG. 2: is a schematic illustration of a laser system according to an embodiment,
- FIG. 3: is a flow chart illustrating a method for detecting and processing information according to an embodiment,
- FIG. 4: is a flow chart illustrating a method for treating a joint according to an embodiment,
- FIG. 5a: is a Structural Illumination Microscopy (SIM) image of an intact cartilage of a minipig joint before a laser treatment,
- FIG. 5b: is a Structural Illumination Microscopy (SIM) image of a cartilage of a minipig joint after a laser treatment, showing a porous structure with pores of 3-10 µm,
- FIG. 5c: is a Structural Illumination Microscopy (SIM) image of a cartilage of a minipig joint after a laser treatment, showing a porous structure with pores of 3-15 µm, the surface of which is covered with calcium ions,
- FIG. 5d: is a Structural Illumination Microscopy (SIM) image of a cartilage of a minipig joint after a laser treatment, showing gas bubbles of 1-2 µm, the surface of which is covered with calcium ions (white rings),
- FIG. 6: is a microscopic image showing a formation of bubbles in cartilage tissue under the action of a laser radiation, with the diameter of an optical fiber being 400 µm,
- FIG. 7: is a graphical representation of a calculated kinetic of the porous structure formation in a laser treated cartilage plate of a horse joint,
- FIG. 8a: is a histological image of minipig articular cartilage in a non-treated joint in 52 days after a laser treatment, showing non-repaired lesions,
- FIG. 8b: is a histological image of a minipig articular cartilage in a treated joint in 52 days after a laser treatment, showing a reparation of the cartilage plate thickness,
- FIG. 8c: is a histological image of the minipig articular cartilage in a treated joint in 52 days after a laser treatment, showing new chondrocytes with multicellular clones and regenerated lamina splendens,
- FIG. 8d: is a histological image of the minipig articular cartilage in a treated joint in 52 days after a laser treatment, showing a zone of affected chondrocytes,
- FIG. 8e: is a histological image of the minipig articular cartilage in a treated joint in 52 days after a laser treatment, showing regenerating chondrocytes with multicellular clones,
- FIG. 8f: is a histological image of the minipig articular cartilage in a treated joint in 52 days after a laser treatment, showing formation of hyaline-fibrous cartilage,
- FIG. 9a: is an electron micrograph of a normal minipig articular cartilage, showing an interterritorial matrix consisting of thin fibrils 15-35 nm in diameter and granular material of proteoglycans,
- FIG. 9b: is an electron micrograph of a regenerated minipig cartilage in 52 days after treatment, showing an interterritorial matrix comprising numerous collagen fibrils, and a long collagen fibril in the middle

### DETAILED DESCRIPTION OF FIGURES AND EXAMPLES

The following description presents examples of the implementation of the present disclosure, and the scope of the present disclosure, but the disclosure is not limited to presented examples. Any variations or replacements can be easily made by persons skilled in the art. Accordingly, the scope of protection of the present disclosure is defined by the attached claims.

Stem cells are crucial for living tissue regeneration and restoration. Stem cells are polypotent cells that can differentiate into different specific cells of human body and restore degenerated tissues and organs. All cells in the living tissues need feeding and breathing, which is provided by blood, but in the avascular tissues like cartilage of the joints and spine or cornea of the eye by water transport of nutrition and oxygen through nature micropore system. In diseased tissues, this system of micropores is blocked, and cells without nutrition gradually fall asleep and then die.

Implantation of external stem cells or specific cells can result in some improvement, but this improvement is always temporary, and the recovery is noncomplete because it does not resolve the problems of the (i) adequate nutrition and (ii) controlling the direction of stem cell differentiation.

The way of the stem cells differentiation is governed by signaling molecules ejected by the existing cells and by the environment, e.g., the surrounding tissue. However, the diseased tissue lacks the signaling molecules, and the degenerated tissue cannot promote correct direction of cell differentiation. That is why, the impregnation of stem cells into the damaged and degenerated cartilage of spinal discs and joints can be beneficial only for an acute damage, but it is usually not effective for chronic osteoarthritis where fibrous tissue is growing instead of hyaline cartilage with the adequate mechanical properties.

Therefore, the stem cell application in medicine requires (i) waking up the sleeping cells; (ii) control over their correct differentiation; and (iii) providing their adequate and permanent feeding. Lasers' use makes it possible to meet all these three challenges.

The laser system according to the present disclosure may be used for:

### (1) Inducing stress waves.

It is known that most of cells are sensitive to the external mechanical load of the specific frequency and amplitude. Specific modulation of the laser source to generate spatially and/or temporally modulated laser radiation can create local heat and mechanical oscillations, which can propagate through a medium and generate the desired mechanical load and/or temperature conditions where the remote stem cell is located. This controls the correct direction differentiation of the remote stem cell.

The modulated laser light may also control a de-differentiation of the mature chondrocytes and other cells. This allows for the rejuvenation of the cellular population of the tissue towards recovery of their ability to divide and restore the lost tissue volume.

### (2) Inducing signaling molecules.

Spatially and temporally modulated laser radiation can act locally and in a focused manner on cells which provide the shock proteins, which are specific signaling molecules, to awaken other dormant cells. Hence, laser radiation allows for activating cells by affecting even a small fraction of the existing cells without damaging and denaturation of the rest cells and of the intercellular matrix.

### (3) Inducing controlled formation of porous structure and propagation behavior.

The total number of activated cells is determined by the propagation distance of the above-mentioned signaling molecules and stress waves. The propagation distance depends on the permeability of the tissue matrix and the sound speed in the matrix, which can be significantly improved by the laser-induced porous structure formation. Thus, the laser-induced porous structure formation is a key factor providing the positive effect of the laser treatment.

### (4) Inducing controlled formation of porous structure and internal stress relaxation.

Internal stress in the tissue or the implant or at their interface may be detrimental to the cells within the matrix. A long-term unrelaxed internal stress may result in the death of the cells. The laser-induced porous structure formation may relax such stress.

### (5) Stabilizing the laser-induced porous structure.

The laser radiation according to the present disclosure also provides a long-term durability of the positive effects, in particular, through stabilizing the created porous structure through a distributed charge on the surface of gas bubbles generated by the laser light. This mechanism enables stabilizing the porous structure created under modulated laser radiation generated by the laser source. A stabilized porous structure reliably promotes the signaling molecules/drug/nutrition transportation.

### (6) Heat control of the laser light.

Overtreatment, in particular, overheating of the tissue may lead to the loss of stability of positive effect or tissue denaturation instead of tissue repair. Therefore, a heat control of the laser light based on a real-time detected information can avoid overheating or over-treatment of the laser.

All the above positive effects of laser treatment can be achieved in the specific range of dosimetry of laser sources and a real-time regulation of the laser source.

Thus, laser activation of stem cells provides
(i) Direct thermomechanical effect on the existing cells, activating their proliferation and synthetic activity.
(ii) Mediated action through specific shock proteins (signaling molecules) emitted by the laser-affected cells.
(iii) Creation of conditions for the rapid propagation of nutrition and signaling molecules over considerable distances beyond the zone of the direct laser exposure, which leads to the activation of the large number of cells and activates their coordinated regeneration activity in large volumes. This leads to the rapid regeneration and growth of tissue of the given composition and properties, for example, regeneration of hyaline cartilage in osteoarthritic joints.

There are very few native stem cells in the degenerated cartilage. Therefore, the pain relief and return to the normal life usually take from three to six months after the laser surgery. The combined use of the stem cell implantation and the laser irradiation using the new laser system will significantly reduce the postoperative recovery time and will make the recovery more complete. What is more, any regular orthopedic surgeon can treat osteoarthritis of the joint with this automatic feedback laser system after about two to three days of training.

### Exemplary System

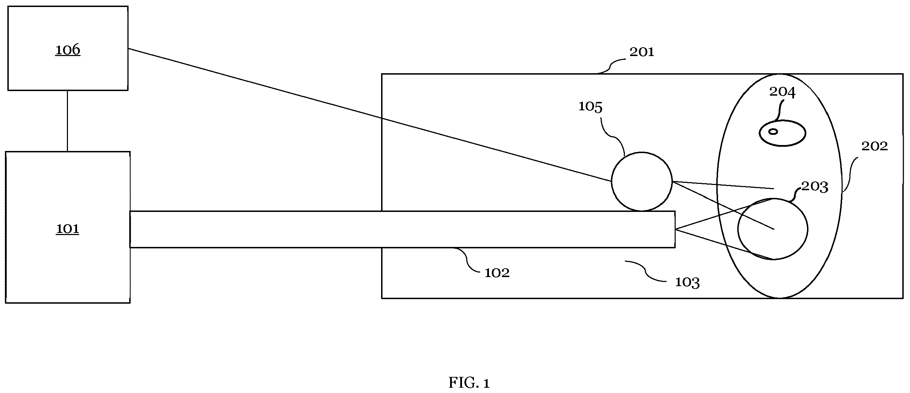

FIG. 1 is a schematic illustration of a laser system disclosed by the present disclosure. The laser system is suitable for a treatment of a cartilage tissue in a joint 201. The laser system comprises: a laser source 101; a feedback controller 106, configured to regulate a dosimetry of the laser source 101 to produce spatially and/or temporally modulated laser light; a first optical delivery element 102, configured to guide the spatially and/or temporally modulated laser light to an area 202 in the joint 201 to irradiate a first part 203 of the area 202; a detecting element 105, configured to detect one or more physical, chemical, mechanical and/or structural characteristics in the area 202 in a real-time, wherein the feedback controller 106 is configured to regulate in a real-time the dosimetry of the laser source 101 based on the real-time detected information pertaining to the one or more physical, chemical, mechanical and/or structural characteristics in the area 202 for a controlled activation of a first stem cell 204 outside the first part 203 of the area 202 to form a hyaline cartilage tissue.

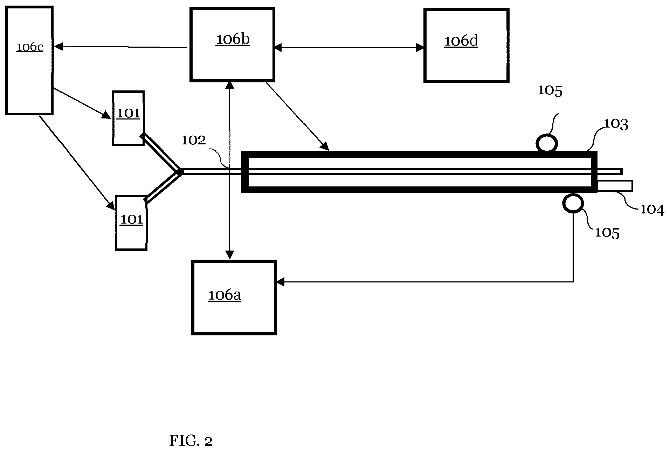

FIG. 2 is a schematic illustration of a laser system according to an embodiment.

The laser system may comprise a diagnostic element 106a, configured to receive a detected information and process the detected information. The diagnostic element 106a comprises a User Interface, configured to present the detected information to a user, e.g., a researcher or a medical doctor. For example, the User Interface may be configured to present a stress distribution and a temperature distribution in the area 202. The diagnostic element 106a may send the detected information unprocessed to a remote high-performance computer, the remote hybrid computational facility, and/or the remote quantum computer 106d. The diagnostic element 106a may further be configured to preprocess the detected information. For example, the diagnostic element 106a may be configured to analyze a detected information pertaining to a scattered light and determine a size distribution of pores in a porous structure.

The laser system may further comprise a feedback control element 106b, configured to manage a data flow in the laser system. The data flow may comprise a flow of real-time detected information pertaining to one or more physical, chemical, mechanical and/or structural characteristics in the area 202; a flow of processed/preprocessed detected information; a command generated for regulating a dosimetry of a laser source 101. The feedback control element 106b may be configured to control the direction and a sequence of the data flow, so that the irradiation of the laser source 101 can be modulated in a real-time based on real-time detected information.

The laser system may further comprise a radiation modulation element 106c, configured to modulate the radiation of the laser 101 source temporally and spatially. The radiation modulation element 106c maybe configured to receive a command generated for modulating the radiation of the laser 101 and regulate a dosimetry of the laser source 101, or the radiation modulation element 106c may be configured to receive the dosimetry value directly from the external high-performance computer, the remote hybrid computational facility, and/or the remote quantum computer 106d.

The laser system may further comprise an external high-performance computer, a remote hybrid computational facility, and/or a remote quantum computer 106d configured to process the detected information or the preprocessed detected information to generate a command for modulating the radiation of the laser source 101 or to regulate a dosimetry of the laser source 101.

The external high-performance computer, the remote hybrid computational facility, and/or the remote quantum computer 106d may be configured to solve equations defining a thermal mechanical problem such as a heat propagation problem, a mechanical problem such as a problem regarding a deformation of a medium deformation. The solution may help to optimize a control of a stress wave.

The external high-performance computer, the remote hybrid computational facility, and/or the remote quantum computer 106d may be configured to solve equations defining a chemical process problem such as a chemical bond breaking problem. The external high-performance computer, the remote hybrid computational facility, and/or the remote quantum computer 106d may be configured to calculate the dynamics of pore shape and size. The solution may help to optimize a controlled formation of porous structure.

The external high-performance computer, the remote hybrid computational facility, and/or the remote quantum computer 106d may be configured to solve equations defining a problem of motion, for example the drift and diffusion of signaling molecules. The solution may help to optimize the transportation of signaling molecules in the area 202.

The external high-performance computer, the remote hybrid computational facility, and/or the remote quantum computer 106d may be configured to calculate dynamics of tissue denaturization. The solution may help to control the temperature and stress in the area 202 to minimize a damage.

The external high-performance computer, the remote hybrid computational facility, and/or the remote quantum computer 106d may be configured to use the solution of the abovementioned inverse problem to establish optimal dosimetry for each step of laser treatment. The calculations maybe conducted within a smalltime interval, for example, within a millisecond up to several minutes, so that the method for treating a joint 201 according to the present disclosure can be carried out continuously.

The diagnostic element 106a, the feedback control element 106b, the radiation modulation element 106c and the high-performance computer, the remote hybrid computational facility, and/or the remote quantum computer 106d may be parts of the feedback controller 106 in FIG.1. Although FIG. 2 shows a separation of the diagnostic element 106a, the feedback control element 106b, the radiation modulation element 106c and the high-performance computer, the remote hybrid computational facility, and/or the remote quantum computer 106d, this separation should not be interpreted as a physical separation but rather a separation of their logical functions. The feedback controller 106 may also refer to a combination of one or more of the diagnostic elements 106a, the feedback control element 106b, the radiation modulation element 106c and the high-performance computer, the remote hybrid computational facility, and/or the remote quantum computer 106d.

For example, if the feedback controller 106 is only configured to process the detected information pertaining to a physical, chemical, mechanical and/or structural characteristics in the area 202, and acquire a property of a stress wave generation, a stress wave propagation, a signaling molecule generation, a signaling molecule transportation, and/or a porous structure formation in the area 202, the diagnostic element 106a alone or a combination of the diagnostic element 106a and the high-performance computer, the remote hybrid computational facility, and/or the remote quantum computer 106d can be seen as a feedback controller 106, then, in this case, the feedback controller 106 facilitates an evaluation of a stress wave generation, a stress wave propagation, a signaling molecule generation, a signaling molecule transportation, and/or a porous structure formation in the area 202 and an initialization of parameters for the laser source 101.

For example, if the feedback controller 106 is further configured to process the detected information in a real-time during the porous structure formation or the stress wave/signaling molecules generation induced by the temporally and spatially modulated radiation of the laser source 101, a combination of the feedback control element 106b and the diagnostic element 106a can be seen as a feedback controller 106, then, in this case, the feedback controller 106 facilitates a monitoring of the laser-induced porous structure formation and activation of the stem cell. For example, a doctor can decide on their own when to interrupt the laser irradiation depending on whether the size distribution of pores in the porous structure reaches a predetermined threshold.

The laser system may comprise a laser 101, configured in a way that its radiation is spatially and temporally modulated by the feedback controller 106. Spatial modulation may refer to varying a location, a shape of the laser beam and the laser-illuminated area and a certain intensity distribution of the laser-induced light in the laser illuminated area. To realize such a spatial modulation, the laser system may comprise one or more lasers sources 101. FIG. 2 only shows two laser sources 101, yet a laser system according to the present disclosure may comprise more laser sources 101.

The laser light delivered by the laser source 101 may be both coherent and non-coherent.

A plurality of lasers 101 may facilitate a complicated spatial modulation of laser irradiation. A spatial modulation may also be realized through a combination of one or more lasers with other auxiliary passive elements, such as lenses, mirrors, an optical splitter, and other optical systems thereof. Each one of the lasers 101 may implement an independent temporally modulated irradiation. A temporally modulated laser irradiation is usually a sequence of pulses of laser irradiation with variable pulse repetition rate, pulse duration, pulse intensities or other variable attributes of a laser pulse. A temporal modulated laser radiation may also refer to non-pulsed laser radiation with a variable shape in the time domain and a variable shape in the frequency domain.

The irradiation of the laser sources 101 maybe real-time modulated. A real-time modulation may include a constantly regulating a dosimetry of the laser source 101, regulating the dosimetry upon receiving a signal from the feedback controller 106 or updating the laser dosimetry after a certain number of pulses in a sequence.

The dosimetry of the laser source 101 is regulated basing on a real-time detected information. For example, the less damage is done to a joint, the more tissue elasticity is required. In this case, basing on the real-time detected information, the feedback controller 106 can determine that a shorter pulse duration is needed for a larger amplitude of a mechanical effect and a larger sequencing frequency of pulses is needed since elastic medium transfers signal at a greater rate. Then the dosimetry of the laser source 101 can be regulated accordingly.

In another example, the joint is more damaged. The feedback controller 106 can determine, basing on the real-time detected information, that the laser pulse should be longer to induce a smaller amplitude of the mechanical effect. In addition, the sequencing frequency of pulses should be smaller since more time is needed for transferring the wave energy. Accordingly, the number of the irradiation series and the number of exposure zones should be increased.

A laser source 101 in the present disclosure may be a combination of several types of lasers, including a solid-state laser (for example a NdYag laser or a Holmium laser) and/or a diode laser.

Each of the laser sources 101 may further be assigned to different tasks. For example, during a treatment, a first laser source 101 may generate modulated laser light to perform heating or modification of a tissue, while a second laser source 101 may generate a modulated laser light to generate stress waves. As another example, the first laser source 101 may generate the modulated laser light for a photochemical effect or another non-thermal effect. The modulated laser light of the first laser source 101 may activate a cell to enhance absorption of other wavelengths, while the second laser source may generate modulated laser light for a controlled formation of a porous structure, or for the redistribution of materials in a nucleus pulpous of a spine disc.

The laser system may further comprise an optical delivery element 102, configured to deliver the modulated laser radiation or laser light to a target. The optical delivery element 102 can be an optical fiber, a bundle of optical fibers, or other types of optical delivery elements. The optical delivery element 102 may also be configured to deliver other laser signals, for example, a probing laser signal for detecting a certain property in a joint 201. In an exemplary embodiment, the imposed laser modulation may account for the possible distortion of the laser signal due to propagation in the laser delivery system 102 and implement corresponding compensations. The optical delivery element 102 may comprise an optical out-coupler for delivering the laser signals in a form of laser irradiation to the target.

The laser system may further comprise one or more detecting elements 105. The detecting elements 105 are configured to detect one or more physical, chemical, mechanical and/or structural characteristics in the area 202 of the joint 201. The one or more physical, chemical, mechanical and/or structural characteristics may comprise a temperature, a stress, the size and the number of pores, types and dimensions of the structural defects, type of collagen and diameter of collagen fibers, thermomechanical characteristics, optical characteristics, electrical characteristics, and other characteristics characterizing the environment in the area 202 and the status of the tissue in the area 202. The characteristics maybe detected in a direct and in an indirect way. For example, the detecting element 105 may comprise a conductivity measuring element, configured to measure a conductivity of a tissue. This characteristic can then be feedbacked as an electrical signal. In another example, the detecting element 105 may comprise an optical receiving element, configured to receive a scattered light. The scattered light can be feedbacked as an optical signal and be processed to deliver the information about a temperature, a stress, a size distribution of gas bubbles, and a size distribution of pores and other structural defects, based on the characteristics of the optical signal such as, for example but not restricted to, wavelength distribution and an angular intensity distribution. In an exemplary embodiment, the detecting element 105 may comprise a conventional diagnostic device, such as one of the following: an X-Ray; a CT; an Ultrasonography (US); an MRI; an OCT; an OCE; a Multispectral Optoacoustic tomography (MSOT); a fluorescence molecular tomography (FMT); and acoustic tomography.

The laser system may further comprise a channel element 103, configured to create an access channel to the to be treated area 202 in the joint 201. In an example, the channel element 103 may comprise a hollow cylinder with a predetermined profile of the cross-section. The hollow cylinder may be a needle. The predetermined profile of the cross-section may be in a form of a circle or an ellipse. The hollow cylinder may be thin walled, to minimize the trauma during the access channel formation. The channel element 103 may be suitable for an introduction of biochemical substances such as stem cells, drug, gas, or signaling molecules.

A gaseous biochemical substance may be a CO₂ gas. A gaseous biochemical may be used to introduce bubbles into the area 202 of the joint 201. The gas bubbles may be configured to assist a stabilized formation of porous structure or a generation of stress wave.

The dosing of such biochemical substances or their introduction may be controlled by the feedback controller 106. The dosing may comprise a composition of liquid and gaseous phases, and/or a chemical or a biochemical substance. The biochemical substance may comprise a liquid or a gaseous phase. The dosing of biochemical substances introduction may comprise a presence or absence of a fluidic medium suction. The dosing of the biochemical substances' introduction may further comprise a parameter such as a velocity or pressure of such fluidic medium suction.

Pressure is administering the fluidic medium. During the laser treatment, the optical delivery element 102 may be introduced to the area 202 of the joint 201 through the channel element 103. In an example, the hollow cylinder may be 5 - 15 cm long and be configured to perform a puncture of the joint 201. Before a puncturing, a control of the positioning of the channel element 103 is performed by the feedback controller 106.

The channel element 103 may be configured to make a fine position adjustment in the joint 201 and record the position of the laser light exerted on the target or a position of the information detected by the detecting element 105, such that a precise spatial modulation of the laser signal and an acquisition of spatial distribution of the detected information is realized. In an exemplary embodiment, the channel element 103 may further comprise and be attached to a servo element for a precise position control.

The laser system may further comprise an effect exerting element 104, configured to exert another chemical, thermal, electrical, magnetic, acoustic and/or mechanical effect in addition to or during the laser irradiation in the area so as to increase the quality of the remote communication effect of the modulated laser light. In an example, the effect exerting element 104 may be a shaft controllably movable within the channel element 103 and capable of simultaneous performing oscillatory movements with a predetermined frequency.

### Exemplary method

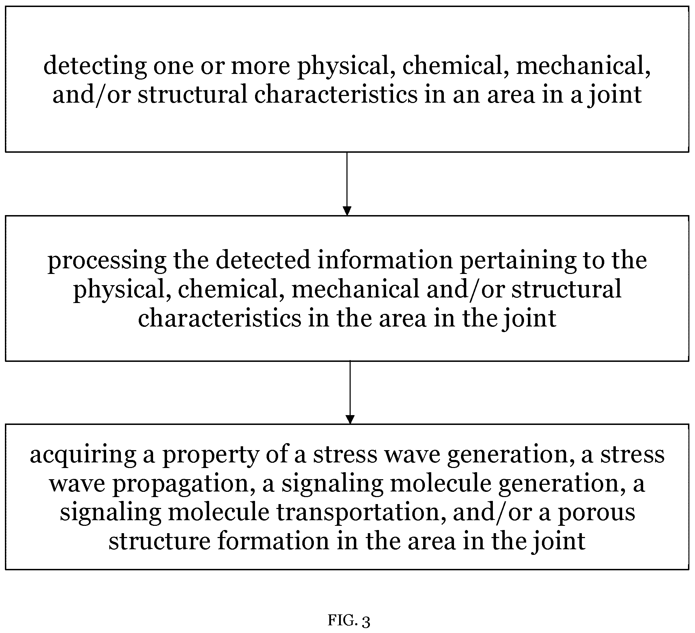

FIG. 3 is a flow chart illustrating a method for detecting and processing information according to an embodiment. In this embodiment, the method comprises:
a) detecting one or more physical, chemical, mechanical, and/or structural characteristics in an area in a joint;
b) processing the detected information pertaining to the physical, chemical, mechanical and/or structural characteristics in the area in the joint; and
c) acquiring a property of a stress wave generation, a stress wave propagation, a signaling molecule generation, a signaling molecule transportation, and/or a porous structure formation in the area in the joint.

As outlined above, the method illustrated in FIG. 3 may be used to evaluate a porous structure, stress distribution, a signaling molecule generation, a signaling molecule transportation, a stress wave generation and/or a stress wave propagation. This structural evaluation maybe carried out for initializing a working condition of the laser. This method may be further used to monitor and evaluate an effect of the modulated laser radiation in the joint. This laser effect evaluation maybe carried out for controlling laser effect or to prevent damage induced by the laser in the joint.

The method illustrated in FIG. 3 may further comprise the step:
d) promoting signaling molecules emitted by laser-activated stem cells, which activate other remote cells to differentiate or dedifferentiate in the desired direction

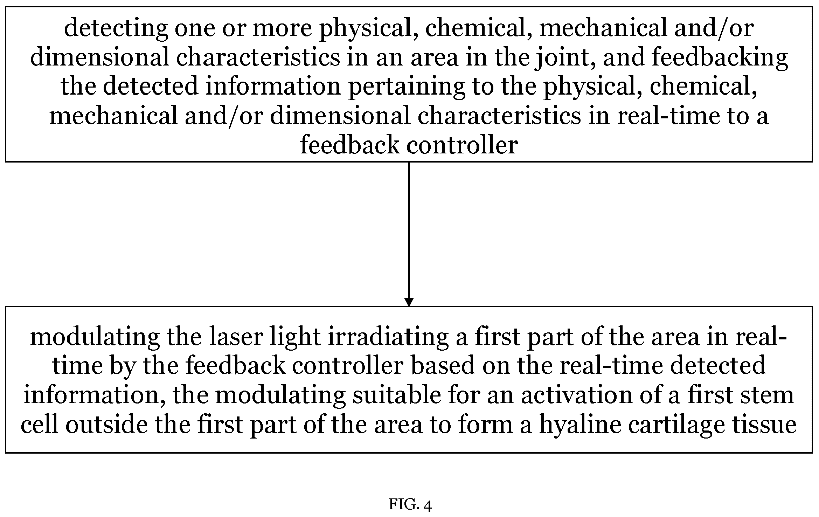

FIG. 4 is a flow chart illustrating method for treating a joint using a temporal and/or spatial modulated laser light comprising a treatment step, wherein the treatment step comprises
a) detecting one or more physical, chemical, mechanical and/or structural characteristics in an area in the joint, and feedbacking the detected information pertaining to the physical, chemical, mechanical and/or structural characteristics in a real-time to a feedback controller; and
b) modulating the laser light irradiating a first part of the area in a joint in a real-time by the feedback controller based on the real-time detected information, the modulating suitable for an activation of a first stem cell outside the first part of the area to form a hyaline cartilage tissue.

In the following an example of the method for treating a joint using a temporal and/or spatial modulated laser light is illustrated. This method corresponds to the method illustrated in FIG. 4 with extra optional steps.

In a first step of the method, an outer skin of the patient is treated with iodine and alcohol and an infiltration of anesthesia, for example, with 2% Lidocaine and % 0.5 Novocain solutions is performed. Then, an access channel is created to the joint. The access channel can be performed through a standard joint puncture using a 5 - 15 cm long 18G.

In a second step of the method, the first optical delivery element 102 is introduced to the to be treated are 202 in the joint 201 and positioned according to the instruction by the feedback controller 106.

In a third step of the method, an initial dosimetry of the laser source 101 is selected based on the real-time detected information from the detecting element 105. The initial dosimetry may be determined based on previous diagnostics or determined on site.

In a fourth step of the method, the area 202 is irradiated based on the real-time detected information to form a porous structure in the area 202 in the joint 201, for example on a cartilage tissue, in a controlled manner.

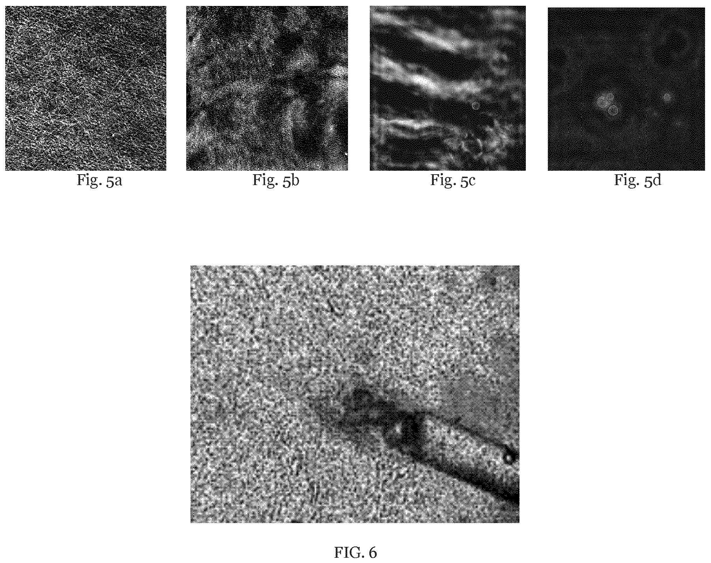

The formation or restoration of the cartilage tissue occurs due to formation of a novel porous structure and/or cleansing and de-clogging of an available but clogged preexisting porous structure in the hyaline plate of the joint. The techniques of the conventional drilling of the cartilage plate and neighboring bone is used to create the pores of tens of microns in diameter. These pores, however, tend to regrow, and, therefore, this effect is temporal. The laser-induced formation of smaller pores of the micron and submicron size makes this effect permanent due to stabilization of small pores by calcium ions. FIG. 5a shows a structural illumination microscopy (SIM) image of non-irradiated cartilage. The pores in the laser-treated cartilage tissue are shown in FIG. 5b-5d. A SIM image of a cartilage of a minipig joint after a laser treatment, shows a porous structure with pores of 3-10 µm, in FIG. 5b. Under the natural mechanical loads, the liquid is extruded from the cartilage plate, and positive ions, calcium and sodium, leave cartilage. The sodium ions move faster, and potassium are left behind and accumulate on the surface of the pores. Repulsion of calcium ions in the small micropores prevent their constriction and stabilizes micropores. Laser-induced pores of 3 µm wide and 15 µm long, the surface of which is covered with calcium ions, are shown in the FIG. 5c.

Stable gas bubbles of the size of 1-2 µm, the surfaces of which are covered with calcium ions (white rings), are shown in FIG. 5d.

In a fifth step of the method, the biochemical substance is introduced into the area 202. The biochemical substance may comprise stem cells or drugs for the treatment. The stem cells may be irradiated by the laser system prior to their introduction to the area 202 in the joint 201, so that the signaling molecules are already generated prior to their introduction to the area 202 in the joint 201.

In particular, the biochemical substance may further comprise: a biochemical substance that influences metabolic processes in a cartilaginous tissue; a biochemical substance modulating the cellular response of a cartilaginous tissue to the external effects; a biochemical substance influencing a histological elements of other kinds of tissue, like nervous, vascular tissues; a biochemical substance purposely modifying a physical and/or chemical property of the cartilage tissue; and/or a biochemical substance exerting the complex effect also on both, biological processes in tissue and on a physical or chemical property thereof.

Examples of such biochemical substances may include: an adrenocortical hormone and analogs thereof such as Dexametasone; a Vitamin such as Vitamin C or a vitamin-like preparation; an Enzyme or an anti-enzyme preparation; an Amino acid; a Macroergic compound or a chemical precursor thereof; a Glucose; an Antioxidant; a Vitreous body or another biogenic stimulant; and/or a Calcium preparation.

The biochemical substance may, in particular, include a signaling molecule such as a growth factor and a cytokine stimulating reparative processes in the cartilage tissue, as well as a corresponding expression inducer and/or synthesis of one or more growth factors or cytokines. Examples for such growth factors or cytokines may include: TGF-β, pDGF, IGF-1, FGF, EGF, EGF, OP-i, BMP-2, BMP-12. These kinds of biochemical substances maybe obtained with cultures of chondrocytes. These kinds of biochemical substances may increase the production of proteoglycanes and the type II collagen, enhance expression of the mRNA aggrecanes, induce an accelerated cellular proliferation, and inhibit their apoptotic death. They may further facilitate a regeneration modification in cartilaginous tissue, such as a rise in the content of proteoglycanes and an appearance of cellular clusters similar to clusters of the normal hyaline cartilage.

The biochemical substance may further include a blocker or competitor of a receptor of the growth factor and the cytokine possessing anti-inflammatory action as well as a biochemical substance suppressing or inhibiting synthesis of the given class of the receptors.

The biochemical substance may further include a substance exerting an effect on ionic permeability of an external or an intracellular membrane of chondrocytes such as a blocker or an activator of an ionic channel.

The biochemical substance may further include a thermolabile precursor of a biologically active substance, such as some metalloproteinases in small doses and with proviso that tissue temperature does not exceed 38°C.

The biochemical substance may further include a photosensitizer, or a biologically active substance conjugated with photosensitizer.

The biochemical substance may further include the first stem cell, or an extract of stem cells. The biochemical substance may further include a complex of surrogate cartilaginous tissue prepared using a tissue engineering method, the complex consisting of stem or chondrogenic cells. The complex may be cultured in vitro on special matrix supports, such as an artificial tissue substitute, with proviso of preserving structural-functional properties of such tissue constructs when passing through the channel element 103.

The biochemical substance may include a drug exerting an effect on nerve endings, or a local anesthetic. The biochemical substance may further include a drug exerting an effect on a granulation tissue vessel growing into a cartilage defect under pathological conditions, the conditions depending on regulators of vascular tone, rheological properties of blood, or vascular wall permeability.

The biochemical substance may include a substance regulating a micro-cavitational process, such as an additive of a surfactant. In other words, the surfactant not only affect pore formation, but promote movement of the signaling molecules through the porous structure. The biochemical substance may include a substance refilling and maintaining a volume of a tissue liquid in the cartilage tissue, such as a drug based on dextran. The biochemical substance may include a weak ionizing or a deionizing solution.

The biochemical substance may include a saline solution for a correction of acid-base, osmotic or ionic state of the tissue, or for a modification of tissue electric conductivity. The biochemical substance may include a drug based on gelatin such as a gelatinol. Such a drug may have effect on an osmotic balance in the tissue, be a source of amino acids for collagens, and/or contribute to a replenishment or substitution of tissue liquid volume.

The biochemical substance may include substances used for the diagnostic purposes, for example, to facilitate the real-time information detection. In particular, the biochemical substance may include the radio-opaque preparation, a substance with fluorescent label, an optically anisotropic substance, such as a substance for the detection of a pathologic tissue modification, a tissue necrosis region, a tissue ultrastructural heterogeneity associated with heterogenic distribution of gas bubbles or pores.

As will be illustrated later, all these biochemical substances, like the signaling molecules, can transport in a large distance due to the modulated laser light, in particular, with the help of the stress wave induced by the modulated laser light. This results in a larger effect area of the biochemical substances mentioned above.

The biochemical substance may be of gaseous or fluidic form. The biochemical substance is delivered to the joint through the channel element 103 forming an access channel to the area 202 in the joint 201. For example, when introducing the channel element 103 to the area 202 of the joint 201, in a lumen thereof guide wire is located which is then removed from the channel element 103 and the optical delivery element 102 is positioned into the channel element 103 in place of it. The optical delivery element 102 may comprise or is attached to a piston, the piston configured to delivers into the area 202 certain amount of biochemical substance. In the case of a gaseous biochemical substance, the gas bubble is disposed close to the end of the channel element 103.

In a sixth step of the method, the area is continued to be irradiated with the predetermined settings combined with an extra effect exerted by the effect exerting element 104 to create thermomechanical zone with controlled spatial and temporal distribution of the stress based on the real-time detected information to generate signaling molecules or a stress wave, the signaling molecules or the stress wave configured to activate a remote stem cell 204.

In particular, the effect exerting element 104 is configured to exert a mechanical oscillation in the area 202. The gas bubbles generated in the fifth step can be separated from the end of the channel element 103 using the modulated laser light and/or the mechanical oscillation. The gas bubble then moves in the area 202 to some distance from the end of the channel element 103. The movement velocity limited by the bubble size is considered in the real-time regulation. The feedback controller then regulates the laser source 101 and the effect exerting element 104 to cause a more efficient distribution of gas in the area 202.

The modulated laser light facilitates an activation of the chondrocytes and biological substances including stem cells by inducing a mechanical effect. The mechanical effects can be caused, in particular, by stress waves arising due to the non-uniform heating waves arising, in turn, as a result of the coordinated rotational oscillations of the water electric dipoles in the cartilaginous matrix or as a result of the stress waves developing due to introduction (and the subsequent movement along it) of the gas or liquid microbubbles into the cartilaginous matrix, as is shown in FIG. 6.

The laser radiation energy is predominantly absorbed by the liquid comprised in intercellular matrix of cartilaginous tissue. The liquid content may be optimized through the porous structure formation in the fourth step. This liquid under the exposure to the laser radiation gets non-uniformly expanded and contracted and breaks the gas bubble into the multitude of smaller bubbles. These microbubbles periodically increase and decrease in volume. The microbubbles are driven to move due to a temperature gradient generated by the modulated laser light into a less heated regions, which is in a direction away from the first optical delivery element 102. This leads to spreading the bubbles in a tissue subject to the described effect and to the increase in the amplitude of pressure waves, as is shown in FIG.6.

The gas, in particular CO2, bubbles arise due to temperature dependence of the gas solubility in the tissue water under the moderate laser heating. A temporal modulation of the laser light provides specific amplitudes and frequencies of the mechanical effect due to oscillations and movement of the gas bubbles. Spatial modulations of the laser light generated by several lasers with the different wavelengths and different penetration depths makes it possible to control the distance of the mechanical wave propagation and, therefore, to treat various targets, in particular, cells and various joint tissues, including cartilage, bone and ligaments and various types of joints including joints in a hip, a knee, and an elbow.

In particular, the bubble generated in a direct thermal mechanical activation and the signaling molecules generated in an indirect biochemical activation maybe combined to further improve the laser treatment effect. For example, when exposed to the laser irradiation, free radical oxidation reactions of oxygen molecules can be activated in a primary bubble in a fluidic medium, for example in an air bubble. If the active oxygen forms and, as a result of the electrostatic interactions with the charged molecules of the intercellular matrix, the surfaces of the air microbubbles can attain an electric charge. Due to electrostatic interactions between the surface of microbubbles and the number of signaling molecules, an increase in the active surface of the intercellular receptor interactions takes place, and the informational tissue metabolism activation, for example the remote control of the remote first stem cell 204 gets improved.

The surface of a gas bubble becomes electrically neutral, which prevents it from lipid peroxidation of cellular membranes. This is realized by occurring binding of the oxygen free radicals to ionized matrix molecules. In addition, it facilitates the transport of the bubbles as well as the molecules interacting with bubble's surface through the porous structure. The molecules can be the signaling molecules or an ion stemming from the drug.

It should be noted that microbubbles emerging in the region exposed to the effect can be formed by different methods. For example, as is outlined above, bubbles can be formed directly during the introduction of a gaseous biochemical substance such as CO₂ or air, through the channel element 102 to the area 202, in particular, irradiated first part 203 of the area 202. The bubbles can also form indirectly upon introducing a non-gaseous biochemical substance, like a tissue material or a fluid substance, promoting a bubble formation in the laser radiated first part 203 of the area 202. The desired effect of the bubble formation can be achieved as a result of the degassing a flowing liquid medium occurring as a result of the electromagnetic radiation of the exposed region. such as the mechanism of the bubble formation illustrated in FIG. 6. In an exemplary embodiment, a non-gaseous biochemical substance promoting the formation of bubbles in the irradiated first part 203 of the area 202 can be administered along the formed access channel.

In conclusion, a controlled formation of the stabilized gas bubbles induced by the modulated laser light based on real-time detected information may facilitate a generation of stress waves, or increase the amplitude of the stress waves, and promote a transportation of the chemicals including the signaling molecules and other biochemical substances and therefore facilitate an activation of the remote first stem cell. This increases the healing effect of each laser treatment step or laser treatment session.

The structure of the cartilage tissue is nonuniform with respect to the distribution of the main components like water, collagen fibers, proteoglycans. The structure of the cartilage tissue is also nonuniform with respect to the thermo-mechanical properties. Therefore, one can distinguish between different domain of a tissue. For example, each domain may comprise regions with a similar orientation of the dipole momentum of water molecules. The displacement of the bubbles and formation of the porous structure occur most easily along the border between different domains. Water molecules can perform rotational oscillations. Oscillation movement of different molecules can interact, for example, to enhance or extinguish each other. The domains with similarly oriented dipole moments can enhance an oscillatory movement and promote a formation of the stress waves which facilitate the activation of the first stem cell.

External effects, such as mechanical, thermal, electrical effects exerted by the effect exerting element 104 can destroy or destabilize the domain structure, which can delay the transformation process. Therefore, the exposure to the external effects is reasonably provided by the several series with the definite time intervals between the series. The intervals are needed to restore the domain structure and to reorganize the porous structure.

During one laser treatment session, the irradiated first part 203 of the area 202 may be sequentially changed. This can be realized through a servo element, for example under ultrasound or OCT control.

In order to accelerate the reorganization process of the porous structure in this time intervals, it is necessary to use a moderate heating, for example, to keep a temperature no more than up to 50°C. This heating can also be useful for inactivating a nerve ending resulting from the cartilage degeneration.

The borders of this additional heating region should be strictly controlled, which is realized through the laser system according to the present enclosure.

While introducing the channel element 103 with a guide wire into the joint, the channel element 103 can be driven into a fluctuating movement which can decrease damage of tissue matrix. The local tissue damage from introducing the channel element 103 may result in the death of a small amount of the cells. The death of the cell also induces a generation of signaling molecules. In other word, the signaling molecule may be generated solely due to a mechanical effect from the guidewire and transported by the stress wave induced by the modulated laser light.

The effect of the method performed by the laser system according to the present disclosure is also reflected in the improved laser-assisted cross-linkage in the collagen fibers. Crosslinking by riboflavin and the blue light or ultraviolet laser has been used previously, however the corresponding therapeutic effect is slow and sometimes leads to side effect in the form of edema. Our approach utilizes the low-intensity UV laser radiation combined with the stem cell injection. This makes the procedure faster, safer, and more complete.

7. In a seventh step of the method, an implant is introduced to the area 202 to heal large defects in the area 202.

8. In an eight step of the method, the stress distribution is detected on the implant, or on the defective tissues, at or in the vicinity between the implant and the defective tissue. The stress may be a mismatch stress between the cartilage tissue and the implant after an introduction of the implant. The stress is then relaxed through forming a porous structure using the modulated laser light.

9. In a ninth step of the method, the laser system is displaced according to an instruction of the feedback controller 106 to another area in the joint 201 and one or more of the above mentioned first to eighth steps are repeated.

10. In a tenth step of the method, a nerve ending in the area 202 of the joint 201 is activated or deactivated on demand using the modulated laser light, thermal, mechanical, or chemical effect of the effect exerting element 104. This may comprise a mechanical removal or decrease in volume of an object compressing a nerve, for example muscular sequester, ligament. The object can be removed by an ultrasonic fractioning, a laser ablation, or other effects. By the deformation thereof, pathological swelling can be removed by a decrease in congestive events. A sustained effect due to change in joint macro architecture by a reconstruction thereof, for example a regeneration of novel tissue, can be improved in this way.

Throughout the method, whenever a laser source 101 or an effect exerting element 104 is used. The method may comprise the following steps to perform the real-time regulation:
a) Measuring the cartilaginous tissue modification characteristics, including the deformity, stress, temperature, structural modification, tissue mechanical and optical properties, electrical impedance,
b) regulating the laser dosimetry or the dosimetry of the effect exerting element 104 based on the measurement parameters, in particular, interrupting or switching off the laser source 101 or the effect exerting element when a predetermined threshold is reached.

It is understood that one or more above mentioned steps are performed simultaneously or in another order different from the numbering.

For example, the fifth step of an introduction of a biochemical substance may be performed prior to, during, or after the fourth or sixth step of irradiation. As another example, the seventh step of introducing the implant can be performed prior to the second to sixth steps.

Each step may also achieve other effects of other steps. For example, during the porous structure formation according to the fourth or the eighth step, the stem cells or other cells may also be activated to generate signaling molecules.

The method or a part of the method may be done repeatedly with the needed number of cycles. The repetition of this method steps promotes an increase in the volume of a gas or other substances delivered into the tissue if removal or spreading of the necessary substances into the joint tissue volume is necessary. A completion of the method is determined basing on the detected information. For example, if a predetermined value is achieved by the detected information. The laser system is removed from the patient's body. Skin around a puncture is treated with iodine and alcohol, a sterile bandage is applied, and patient is brought to a ward.

In addition to the effects outlined above, the modulated laser light may have other extra positive effects on a cartilage tissue.

For example, it may promote a release of the intracellular Ca²⁺ and, as a sequence, facilitate a renewal or rejuvenation of a cellular population as a result of the controlled apoptotic death of inactive chondrocytes.

As another example, it may promote an increase in the functionally active surface of the cellular membranes and enhance the intercellular interactions.

A combination of all possible positive effects of the modulated laser light causes substitution of the pathologically modified joints sites or articular cartilage with young or newly formed cartilaginous tissue, i.e., it causes an improved regeneration of the damaged or lost structures over the prior art. The newly formed hyaline cartilage in joints fills up pathologically modified areas of the articular surfaces occurring, as a result, of the degenerative or traumatic diseases, for example, osteoarthroses, chondromalacia, and joint traumas. This provides the clinically significant restoration of the functional joint activity.

### Exemplary algorithm

As outlined above, the modulated laser light used in the present disclosure can alter the thermal dynamic parameters in the area of the joint. Most serious alterations in the parameters can be due to structural and phase transformation occurring in the tissue during laser irradiation. In particular, the non-linearity of the math problems makes it difficult to provide precise calculations in a real-time using existing medical devices associated with the regular computers inside. A remote high-performance computer provides the precise calculations with regard to these alterations.

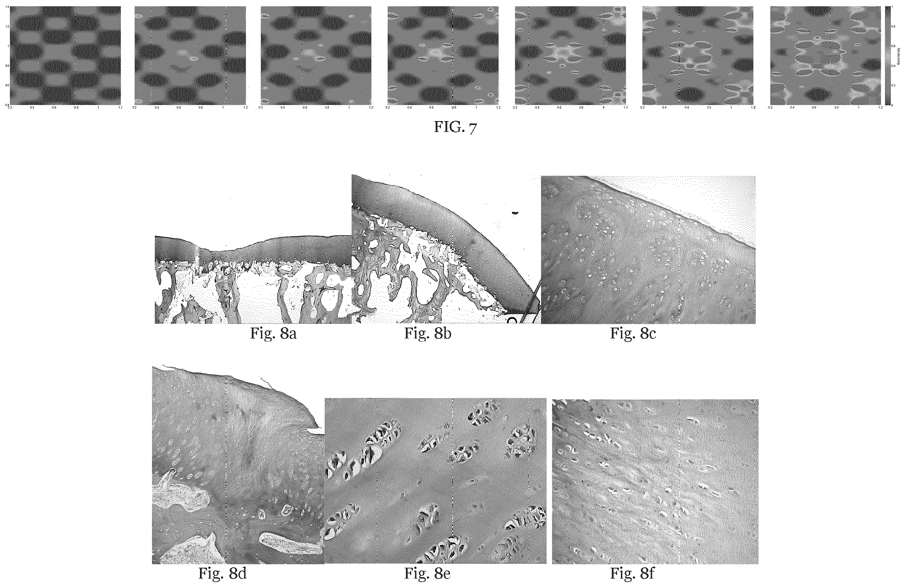

An exemplary algorithm can be characterized through the mathematical problems and sub-problems that need to be solved by the feedback controller 106 for a real-time regulation of the laser system. The mathematical problems, sub-problems and tasks may include:
1. A 3D thermomechanical problem considering thermal expansion for various three-dimensional bodies.
2. The 3D kinetics of laser-induced bond breaking with allowance for partial bond restoration.
3. An accurate calculation of the laser heat source based on the problem of propagation and absorption of light in different structures of the joints.
4. An adequate calculation of the laser heating. The 3D non-stationary heat problem for the spatial and temporal modulation of the laser heat source, considering both linear and non-linear terms with regard to laser-induced phase transformation of tissues such as a denaturation of tissues induced by the laser light.
5. Kinetics of the porous structure formation, including a branching and merging of pores. FIG. 7 demonstrates an example of the pore size kinetics calculated in a real-time.
6. Kinetics of the tissue denaturation and a calculation of the range of safe laser dosimetry to minimize a tissue denaturation.

An algorithm used in the present disclosure should solve the inverse problem of determining the dosimetry of laser source 101, the dosimetry of the effect exerting element 104 and/or the dosimetry of biochemical substance introduction to achieve a positive effect, such as desired stress wave generation, and minimizing the negative, such as overheating.

### Examples of successful treatments

The present disclosure has been implemented in certain preliminary experiments disclosed below. Although the disclosure has been implemented in these examples, these examples may contain extra steps, which should be not seen as restrictive to the present disclosure.

### First example

A 2-year-old minipig underwent surgery under ketamine anesthesia after sedation with romifidine. On the cartilage of the medial femoral condyle, two defects 4 × 6 mm in size and 0.5 mm deep were created. Ligaments in the knee joints of both hind limbs (left and right) were damaged (partially cut)._After the surgical creation of defects, the animal was forced to move on the joints for 56 days. On the 57th day, the left joint was opened, and the right joint was left as a control. Visual observation showed that the surgically created defect did not change its size, and on the surface of the cartilage in the zone of maximum mechanical load during movement, an additional secondary defect of irregular shape and larger sizes (about 7 ×15 mm) appeared at the distance of about 2 mm from the primary defect. Severe edema was observed around the damaged ligament.

The left joint was treated with the following protocol:
Firstly, diagnostic parameters, such as the elastic modulus, and the electrical impedance were measured with the feedback control system using optical coherent elastography and electrical measurements to establish initial dosimetry for laser treatment. The first set of the laser treatment was performed to create a porous structure in the cartilage plate and periosteum using the laser device including a 1440 nm diode laser source 101, a detecting element 105 configured to perform OCE and impedance measurements and a feedback controller 106 for a real-time regulation. The initial dosimetry of the laser source comprised the power of 0.8 W, the pulse duration of 200 ms, the pulse repetition rate of 2.5 Hz, ten pulses in a series and the 6 s period between series. The feedback controller 106 stopped irradiation when the preset parameters of elasticity module and electrical impedance were achieved. At the second step, the damaged zone of the ligament was hydrated with 0.1% riboflavin and irradiated with a laser beam with wavelength 368 nm (which corresponds to one of the riboflavin light absorption peaks), the illuminating diameter of 7 mm for 20 s at ~10 mW/cm² to create an additional strengthening cross-linkage between the collagen fibers in the ligament. At the third step the defect was hydrated with marrow aspirated from the sternum of the animal and at the fourth step the cartilage at the boundary of the secondary defect was irradiated with the laser light with the wavelength of 1440 nm, the initial laser power of 0.5 W, the spot diameter of 0.6 mm, the distance between the laser spots of 3 mm, the pulse duration of 70 ms, the pulse repetition rate of 1 Hz, 8 pulses in a series, period 5 s between series. The laser power during the treatment was controlled by a remote high-performance computer based on the light scattering characteristics measured in a real-time by the detecting element 105 to maintain the amplitude of the oscillation pressure in the range of 5-15 kPa. The total exposure time for each spot was also controlled by the feedback controller that stopped irradiation when the specified parameters were reached.

Fifty-two days post treatment after the animal was sacrificed, and visual and morphological analysis was performed. It was found that on the treated joint, (i) both irradiated (secondary) and non-radiated (primary) lesions were covered with a wight cartilage-like tissue; (ii) the ligament looks almost normal without visible edema; (iii) the covering of the lesions was more complete and about three times faster than that was achieved by using laser-induced regeneration without additional cross-linkage formation and the stem cell impregnation and activation.

FIG. 5 demonstrated the Structural Illumination Microscopy (SIM) images showing the formation of a porous structure and gas bubbles in laser treated cartilage plate.

Slices of tissues (4-5 µm thick) were made using Cryomicrothome Leica CM1900 at -15 °C with refrigerant Jung Tissue Freezing Medium. The marker Fluo-4 Ca dye (Thermo Fisher Scientific, USA) was applied to detect Ca2+ ions. Images were obtained with the superresolution microscope, the optical microscopy experimental (OMX) system v3.0 (Applied Precision, Inc., the GE Healthcare company). The 532nm laser was used for the fluorescence excitation, and the objective immersion oil with the refractive index of 1.514. Super resolution fluorescence images were recon- structed using softWoRx 2.0 (Applied Precision, Inc.) with the raw data collected with OMX. Reconstructed images were post-processed and displayed using ImageJ.

Histological analysis demonstrated the prevalence of the hyaline-type and fibrous-hyaline cartilage in the zones of both (primary and secondary) damages with reparation of lamina splendens (LS), which was previously never achieved using any other techniques. FIG. 8 demonstrates the histological images of minipig articular cartilage, damaged and treated zones in 52 days after the treatment.

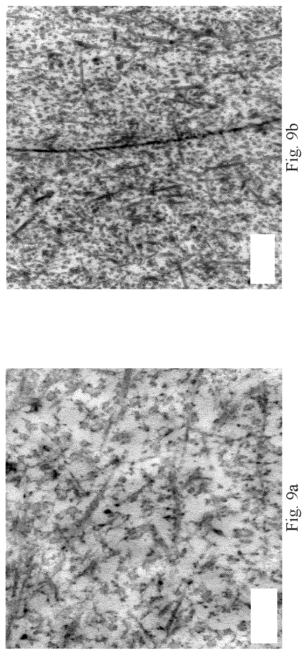

Although the LS is of great importance to provide lubrication and mechanical properties of articular cartilage, it is never restored after the replacement of the damaged areas with the fibrous cartilage or fibrous connective tissue. The restoration of the LS is the important evidence of an advanced character of regeneration under the laser radiation in combination with the injection of marrow stem cells. Therefore, this example clearly demonstrated that the combination of the different real-time modulated laser light, biochemical substances based on the real-time detected information detected from different targets (cartilage plates, stem cells, ligaments), results in a laser-induced regeneration of the cross-linkages due to the activated stem cells. This makes it possible a fast and more complete reparation of the joint with the prevalence of the hyaline cartilage. FIG. 9 demonstrates the electron micrographs of the articular cartilage growing in the treated lesion. The untreated joint (shown in FIG. 8a) kept its damaged condition with osteoarthritic zones and necrotic tissues. It should be noted that a simple laser treatment have led to the restoration of the damaged joints in minipigs within six months (which is 3 times longer than in this example).

### Second example

A 64-year-old patient suffered from pain in the knee joints for 6 years. Over the past 1.5 years, there have been periods of the prolonged exacerbations. The X-ray revealed the signs of deforming arthrosis of the knee joint. The MRI revealed changes in the hyaline plates, the latter are thinned and have a pathological "fringe". The diagnosis included gonarthrosis and femoral-patellar arthrosis stage II, damage to the medial meniscus stage III, and lateral meniscus III Art. according to Stoller, subtotal rupture of the anterior cruciate ligament, damage to the lateral collateral ligament, degenerative changes in the medico-collateral ligament. Thinning and rupture of the medial collateral ligament, swelling of the perifocal tissue. Baker's cyst (73 × 14 mm).

The treatment was performed in several stages. Under the local anesthesia with lidocaine and bupivacaine, arthroscopy of the knee joint was performed with the injection of a sufficient volume of saline. A liquid outflow channel from the knee joint was created. The first optical delivery element 102, in this case an optic light guide, was introduced through the channel element 103, in this case an endoscope.

Laser irradiation of surfaces coated with fringe was carried out with the following parameters: the radiation wavelength of 2.09 µm, the pulse duration of 500 µs, the pulse sequence frequency of 10 Hz, the irradiation series duration of 10 seconds, the interval between irradiation series of 10 seconds, the initial power of 3 W, and the total time of irradiation of 8 minutes.

The laser power during the treatment was controlled by the remote high-performance computer 106d based on the light scattering and speckle dynamics measured in a real-time by the detecting element 105. The feedback controller 106 regulated the system to maintain the amplitude of the oscillation pressure in the range of 5-15 kPa. The total exposure time and the smoothing of the fringe was controlled using the feedback controller 106. Pulsating flow of liquid (normal saline) through the knee joint region was maintained during the irradiation. At the next stage of the treatment, the 5 ml of bone marrow aspirate was injected into the joint. At the last stage of the treatment, the high-frequency current source was introduced into the joint, the articular surface and the cartilaginous plate were irradiated with the following parameters: the frequency of 2 MHz, the exposure series duration of 5 seconds, the interval between series of 5 seconds. The radiation power was controlled using the feedback controller 106 based on light scattering and optoacoustic temperature measurement. The temperature in the impact zone reached 42°C and was maintained at this value with the accuracy of ±0.3°C for 25 seconds for each impact zone. During exposure, the flow of fluid (physiological saline) was maintained through the region of the hyaline plates and along the articular surface. A total of four zones were treated.

In the post-treatment period, there was a significant improvement. Three weeks after the treatment, there was a significant decrease in pain when walking. Pain completely stopped after two months. The MRI in 12 months showed the thickening of the cartilaginous plate, the significant reconstruction of the posterior cruciate ligament and the partial reconstruction of the anterior cruciate ligament, with no perifocal edema, the 40% reduction in the size of the Baker's cyst. The patient returned to the normal life and activity. This case have demonstrated the fast pain relief and significant reparation (supported by the MRI evidence) of the knee joint, including cartilage plate and ligaments.

### Third example

A 59-year-old patient suffered from the back pain syndrome more than 8 years with the regular back pain attacks. Exacerbations continued up to several months. Conservative treatment was in a majority of cases successful. Sanatorium-resort treatment was regularly carried out during the last 4 years. Last year leg pains drastically increased, a neurogenic intermittent claudication syndrome developed.

Using the MRI, the picture of the old lumbar spine spondyloarthrosis was observed, with the formation of the spinal channel stenosis at the level of L3-L4, L4-L5 and L5-S1. The L3-L4 disc protrusion at the level of L3-L4 and bilateral hypertrophy of the yellow ligaments was the main cause of the stenosis. The spinal channel size was 8 mm. At the level of L4-L5, the stenosis was connected with the coarse compression of the dural sac, a central 8 mm disc hernia and hypertrophied joint and the smooth ligament. The spinal channel size was 5 mm.

The CT detected "the vacuum effect" at this level. At the level of L5-S1, the spinal channel stenosis was caused by the disc protrusion with the signs of retrolysthesis and the coarse hypertrophy of the yellow ligament at this level. The signs of articular hyperplasia and hypertrophy were seen. The spinal channel size at this level was 9 mm.

Under general anesthesia, the treatment was done in several steps:
A micro disc compression at the level of L3-L4, L4-L5 and L5-S1 was performed. Compression of the dural sac and radices by the yellow ligament and hypertrophied sections of the facet joints was eliminated at all levels.

Then manipulations on the discs and vertebral joints were done.

The L3-L4 disc: puncture of the disc was done, tension in the disc was absent. Discography showed a rupture of the anulus fibrosis in the zone of the left lateral disc sections and paravertebral penetration of the radio-opaque substance. 0.5 cm³ of the chondroitin sulphate-based gel was injected into the disc cavity. Then the light guide was introduced into the disc (though the same needle) and irradiation in 5 zones was performed with laser wavelength of 1.56 microns. The irradiation time of one zone was determined automatically using the feedback controller 106 which switched off radiation in one second after the occurrence of the turbulent flows in the exposure zone. An occurrence of the turbulent flows was recorded using the caking picture dynamics. Radiation power was at the beginning set to 0.7 W and then it automatically changed using the feedback controller 106 which switched on temperature measurement such that temperature in the exposure zone was maintained at 46°C with accuracy of ± 0.5°C.

The L4-L5 disc: a free sequester (2 cm in size) was removed from below the longitudinal ligament. The disc cavity was washed with NaCl solution (0.5%). An endoscope revision revealed a large defect in the hyaline plate in the central zone of the L5 vertebral body and destruction of the annulus fibrosus throughout the disc. Laser irradiation of the disc in 6 zones was done.

The laser with the wavelength 1.32 microns was used. The zone irradiation time was determined automatically using the feedback controller 106 which switched off the radiation after appearance of the porous structure (micro pores) in the exposure zone. The appearance of the micropores was registered using an optic coherent tomography. Then the biological tissue suspension (stem cells taken from the same patient in sternal puncture) was injected into the disc cavity.

The L5-S1 disc: following removal of fragments of the annulus fibrosus from both sides and formation of a bed, B-Twin cages were introduced into the disc cavity. Thereafter, the laser irradiation of the remaining disc elements was performed on both sides in the three right zones and the three left zones. The facet joints of the L3-L4, L4-L5 and L5-S1 were also sequentially exposed to laser irradiation with laser wavelength 1.44 microns following puncture and introduction of air. Irradiation time of one zone was determined automatically using the feedback controller 106 which switched off irradiation in 2 seconds after formation of micro bubbles in the exposure zone. Formation of micro bubbles was recorded using the acoustic transducer. Radiation power was at the beginning set to 1 W and then automatically changed using the feedback controller 106 which switched on optoacoustic measurement of temperature such that temperature in the exposure zone was maintained at 42°C with accuracy of ± 0.3°C.

During the post-treatment period, a significant improvement was noted: leg pains in loading disappeared, the lumbar pain syndrome decreased. Limiting physical load and wearing a corset was recommended for 2.5 months post laser surgery. During this period, exacerbations of pains were not observed. Examination in 6 months and in one year showed an absence of the spondyloarthrosis signs in the L3-L4, L4-L5 (MRI) and no signs of joint hypertrophy and destruction were seen.

### Fourth example

The nine-year-old Irish Sporthorse gelding acutely 3/5 lame on the LH starting after fall in turnout. There was moderate osteophytosis at the medial aspect of the left hind limb fetlock joint, with an angular osseous fragment at the mediodorsal aspect of the P1 articular margin. The osseus density of the mediodistal aspect of the left 3rd metatarsus was slightly heterogeneous with focal radiolucency. There was moderately increased soft tissue opacity within the fetlock joint. There were multiple pinpoint mineral opacities superimposed on the soft tissue plantar to the pastern joint. The diagnosis showed (i) Moderate osteoarthritis of the fetlock of the left hind limb with synovitis/articular effusion; (ii) a large (20 × 30 mm) defect in the cartilage plate; (iii) tendon and ligament injurie.

Under ultrasound imaging and ketamine anesthesia after sedation with romifidine, the treatment was performed in several stages. First, the horse was injected in the actual joint with the pro-stride (autologous conditioned plasma). Then the laser treatment was performed by the laser system using two laser sources 101 with wavelengths of 720 nm and 1320 nm, the pulse duration of 500 ms and 20 ms, and pulse repetition rate of 0.25 Hz and 2 Hz respectively. At the beginning, the radiation power was set to 2 W and then changed using the feedback controller 106 based on optoacoustic and light scattering measurements. The laser treatment was performed in offline control using a predefined settings table for laser setting which provided desired space distribution of the porous structure (micropores) enhancing the water permeability and a feeding of the cells (See the FIG. 7). Three series of laser pulses with six pulses in a series, and 5 s interval were applied for each zone around the lesions. Twenty zones of elliptical shape with distance between zones of 3.5 mm were treated.

At the next stage, the liquid and fast-hardening implant made of silk fibroin and hyaluronic hydrogel with stem cells was introduced into the lesion. After 12 minutes, the laser treatment was performed using the laser radiation with the wavelength of 1560 nm, the pulse duration of 200 ms, the pulse repetition rate of 0.5 Hz, to relax the mismatch stress at the implant-cartilage interface. The laser power and exposure time was controlled by the feedback controller 106 measuring temperature with optoacoustic and residual stress with optical coherent elastography.

During the post-treatment period, a significant improvement was noted. The lameness improved significantly 40 days after treatment and almost disappeared after two and a half months. According to the CT data, there were no signs of osteoarthritis and articular effusion, the almost complete absence of osteophytes, and the significant decrease in calcification of the synovial membranes. The MRI demonstrated the good engraftment of the implant and almost complete restoration of the tendon and ligament. So, in three months a significant improvement has been achieved including in areas adjacent to the zones of laser exposure.

The description of the specific embodiments and the Figures merely serves to illustrate the techniques of the present disclosure and the advantageous effects associated therewith but should not imply any limitation. The scope of the disclosure is to be inferred from the appended claims.

### List of reference signs

- 101: laser source
- 102: optical delivery element
- 103: channel element
- 104: effect exerting element
- 105: detecting element
- 106: controller
- 106a: diagnostic element
- 106b: feedback control element
- 106c: radiation modulation element
- 106d: remote ultra-fast computer
- 201: joint
- 202: area
- 203: first part
- 204: first stem cell

## Claims

1. A laser system suitable for a treatment of a cartilage tissue in a joint (201), the laser system comprising:
a laser source (101);
a feedback controller (106), configured to regulate a dosimetry of the laser source (101) to produce spatially and/or temporally modulated laser light;
a first optical delivery element (102), configured to guide the spatially and/or
temporally modulated laser light to an area (202) in the joint (201) to irradiate a first part (203) of the area (202); and
a detecting element (105), configured to detect one or more physical, chemical,
mechanical and/or structural characteristics in the area (202) in a real-time;
wherein the feedback controller (106) is configured to regulate in a real-time the dosimetry of the laser source (101) based on the real-time detected information pertaining to the one or more physical, chemical, mechanical and/or structural characteristics in the area (202) for a controlled activation of a stem cell (204) outside of the first part (203) of the area (202) to form a hyaline cartilage tissue.

2. The system of claim 1, wherein:
the feedback controller (106) is configured to regulate the dosimetry of the laser source (101), so that the generated modulated laser light changes a temperature and/or a stress of a tissue in the first part (203) of the area (202) in a specific sequence and/or simultaneously.

3. The system of claim 1 or 2, further comprising:
a channel element (103), configured to create an access channel to the area (202) in the joint (201);
wherein the channel element (103) is configured to deliver a pre-activated stem cell to the area (202), and
wherein the first optical delivery element (102) is configured to guide the spatially and/or temporally modulated laser light through the channel element (103) to the first part (203) of the area (202).

4. The system of one of the preceding claims, wherein:
the feedback controller (106) is further configured to control in a real-time, based on the real-time detected information, a position of the first optical delivery element (102) in the area (202) during the irradiation.

5. The system of one of the preceding claims, wherein:
the feedback controller (106) is further configured to regulate the dosimetry of the laser source (101) for a controlled formation of a porous structure on the cartilage tissue and/or another object in the area (202) based on the real-time detected information.

6. The system of one of the preceding claims, wherein:
the first optical delivery element (102) is configured to irradiate the first part (203) of the area (202) to induce a formation of a cross-linkage of tissue between an implant and the cartilage tissue in the area (202).

7. The system of claim 6, wherein:
the detecting element (105) is configured to detect a stress distribution at or in the vicinity of an interface between the implant and the cartilage tissue, and
the feedback controller (106) is configured to control the laser source (101) and the first optical delivery element (102) to form the porous structure at or in the vicinity of the interface between the implant and the cartilage tissue according to the detected stress distribution.

8. The laser system of one of the preceding claims, further comprising:
an effect exerting element (104), configured to exert a thermal, electrical, magnetic and/or mechanical effect on a tissue in the area (202), wherein the feedback controller (106) is configured to regulate the effect exerting element (104) in a real-time based on the real-time detected information.

9. The laser system of one of the preceding claims, wherein:
the feedback controller (106) is configured to regulate the laser source (101) and/or
the effect exerting element (104) to activate or deactivate a nerve ending in a real-time based on the real-time detected information.

10. The laser system of one of the preceding claims, wherein:
the feedback controller (106) comprises and/or is coupled to a remote high-performance computer, a remote hybrid quantum-classical computational facility, and/or a remote quantum computer (106d); and/or
the feedback controller (106) comprises and/or is connected to a storage device, the storage device storing an offline settings table, wherein the settings table is calculated by a remote high-performance computer, a remote hybrid quantum-classical computational facility, and/or a remote quantum computer (106d).

11. A method for detecting and processing information, comprising:
a) detecting one or more physical, chemical, mechanical and/or structural characteristics of a tissue in an area (202) in a joint (201);
b) processing the detected information pertaining to the physical, chemical, mechanical and/or structural characteristics of the tissue in the area (202) in the joint (201); and
c) acquiring a property of a stress wave generation, a stress wave propagation, a signaling molecule generation, a signaling molecule transportation, and/or a porous structure formation in the area (202) in the joint (201) in a real-time during the stress wave generation, the signaling molecule generation, and/or the porous structure formation,
d) promoting an interaction between a cell in an irradiated part (203) of the area (202) and a cell (204) outside the irradiated part (203) of the area (202).

12. The method of claim 11, wherein
the processing the detected information comprises:
generating a value for a dosimetry of a laser source (101) in a real-time based on the detected information pertaining to the physical, chemical, mechanical and/or
structural characteristics in the area (202) in the joint (201);
wherein, optionally:
the stress wave generation, the signaling molecule generation and/or the porous structure formation is induced by a temporally and/or spatially modulated laser light generated by the laser source (101).

13. The method of claim 12, wherein
the detecting of the physical, chemical, mechanical and/or structural characteristics in the area (202) in the joint (201) comprises:
detecting a temperature in the area (202) in the joint (201), wherein the value for the dosimetry of the laser source (101) is generated if the temperature is within a predetermined range.

14. The method of one of claims 11 to 13, further comprising:
acquiring a property of a formation of a cross-linkage of a tissue in a real-time during the cross-linkage formation.

15. The method of claim 14, further comprising:
detecting stress distribution at or in the vicinity of an interface between the cartilage tissue and an implant, for changing the stress at or in the vicinity of the interface between the cartilage tissue and the implant to reach a predetermined value.
